# EUROPÄISCHE PATENTSCHRIFT

(11) **EP 2 090 605 B1**
(45) Veröffentlichungstag und Bekanntmachung des Hinweises auf die Patenterteilung: **13.07.2011**
(21) Anmeldenummer: 09001364.0
(22) Anmeldetag: 31.01.2009
(51) Int. Cl.: C08G 64/24, C07C 68/02

(54) **Verfahren zur Herstellung von Polycarbonaten**
Method for making polycarbonates
Procédé de fabrication de polycarbonates

(30) Priorität: 13.02.2008 DE 102008008841; 16.08.2008 DE 102008038031
(43) Veröffentlichungstag der Anmeldung: 19.08.2009
(73) Patentinhaber: Bayer MaterialScience AG, 51368 Leverkusen (DE)
(72) Erfinder: Heuer, Helmut-Werner, Dr., 47829 Krefeld (DE); Ooms, Pieter, Dr., 47800 Krefeld (DE)

(56) Entgegenhaltungen:
- EP-A- 0 070 529
- EP-A- 1 249 463
- DE-A1- 3 717 057
- DE-A1- 4 220 239

## Beschreibung

Gegenstand der vorliegenden Erfindung ist ein Verfahren zur kontinuierlichen Herstellung von Polycarbonaten und Diarylcarbonaten nach der Methode des Phasengrenzflächenverfahrens bei dem sowohl die Mischung der organischen und wässrigen Phase als auch der vorgelagerte Oligomerisierungsschritt bzw. die Bildung des Chlorameisensäureesters der Monoarylverbindung in einer speziellen Pumpe erfolgt.

Die Polycarbonatherstellung nach dem Phasengrenzflächenverfahren ist bereits von Schnell, "Chemistry and Physics of Polycarbonates", Polymer Reviews, Volume 9, Interscience Publishers, New York, London, Sydney 1964, S. 33-70; D.C. Prevorsek, B.T. Debona und Y. Kesten, Corporate Research Center, Allied Chemical Corporation, Morristown, New Jersey 07960: "Synthesis of Poly(ester Carbonate) Copolymers" in Journal of Polymer Science, Polymer Chemistry Edition, Vol. 18, (1980)"; S. 75-90, D. Freitag, U. Grigo, P.R. Müller, N. Nouvertne', BAYER AG, "Polycarbonates" in Encyclopedia of Polymer Science and Engineering, Volume 1 1, Second Edition, 1988, S. 651-692 und schließlich von Dres. U. Grigo, K. Kircher und P. R- Müller "Polycarbonate" in Becker/Braun, Kunststoff-Handbuch, Band 3/1, Polycarbonate, Polyacetale, Polyester, Celluloseester, Carl Hanser Verlag München, Wien 1992, S. 118-145 beschrieben worden.

Des Weiteren wird auch in EP-A 0 517 044 oder EP-A 520 272 das Phasengrenzflächenverfahren zur Herstellung von Polycarbonat beschrieben:

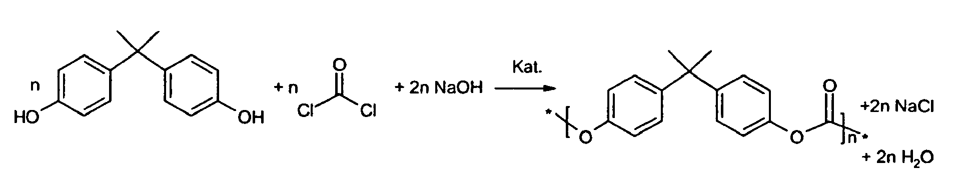

Zur Herstellung von Polycarbonat nach dem Phasengrenzflächenverfahren erfolgt die Phosgenierung eines in wässrig-alkalischer Lösung oder Suspension vorgelegten Dinatriumsalzes eines Bisphenols oder eines Gemisches verschiedener Bisphenole in Gegenwart eines inerten organischen Lösungsmittels oder Lösungsmittelgemisches, welches neben der wässrigen eine zweite organische Phase ausbildet. Die entstehenden, hauptsächlich in der organischen Phase vorliegenden, Oligocarbonate werden mit Hilfe geeigneter Katalysatoren zu hochmolekularen, in der organischen Phase gelösten, Polycarbonaten aufkondensiert, wobei das Molekulargewicht durch geeignete Kettenabbrecher (monofunktionelle Phenole) kontrolliert werden kann. Die organische Phase wird schließlich abgetrennt und das Polycarbonat durch verschiedene Aufarbeitungsschritte daraus isoliert.

Die Durchführung der Polycarbonatsynthese sowie der Diarylcarbonatsynthese kann kontinuierlich oder diskontinuierlich geschehen. Die Reaktion kann in daher in Rührkesseln, Rohrreaktoren, Umpumpreaktoren oder Rührkesselkaskaden oder deren Kombinationen erfolgen, wobei durch Verwendung der bereits erwähnten Mischorgane sicherzustellen ist, dass wässrige und organische Phase sich möglichst erst dann entmischen, wenn das Synthesegemisch ausreagiert hat, d.h. kein verseifbares Chlor von Phosgen oder Chlorkohlensäureestern mehr enthält.

Der erste Schritt zum Aufbau der Oligocarbonate wird gemäß Stand der Technik beispielsweise in einem Umpumpreaktor durchgeführt; siehe z. B. EP 1 249 463 A1, US 2004/0158026 A1, US 6,613,868 B2. Im Umpumpreaktor erfolgen dabei das Mischen des eingebrachten Phosgens mit ebenfalls eingebrachtem Dinatriumsalz eines Bisphenols (oder eines Gemisches verschiedener Bisphenole) sowie die ersten Oligomerisierungsschritte. Entstehende Chlorformiatgruppen reagieren dabei mit vorliegenden Phenolatendgruppen zu wachsenden Oligomeren, die unterschiedliche Endgruppen (Phenolat oder Chlorformiat bzw. aus beiden gemischte Spezies) enthalten. Ein gewisser Anteil einbrachten Bisphenols befindet sich noch unreagiert im Gemisch. Zwar weist der Umpumpreaktor dahingehend Vorteile auf, dass Dosier- oder Konzentrationsschwankungen nicht ungedämpft bis zum Endprodukt durchschlagen können, sondern vielmehr durch Rückvermischung bei eingestelltem Umpumpverhältnis zu Zu- bzw. Ablauf der Reaktionsmischung eine Äquilibrierung von Schwankungen erreicht wird. Jedoch besteht ein signifikanter Nachteil darin, dass für großtechnisch vorteilhafte Parametereinstellungen relativ breite Molekulargewichtsverteilungen entstehen. Ein weiterer Nachteil ist darin zu sehen, dass Umpumpreaktoren relativ voluminöse Baugruppen darstellen.

Die Herstellung von Diarylcarbonaten (wie z. B. Diphenylcarbonat) erfolgt üblicherweise durch ein kontinuierliches Verfahren, durch Herstellung von Phosgen und anschließende Reaktion von Monophenolen und Phosgen in einem inerten Lösungsmittel in Gegenwart von Alkali und einem Stickstoffkatalysator in der Grenzfläche.

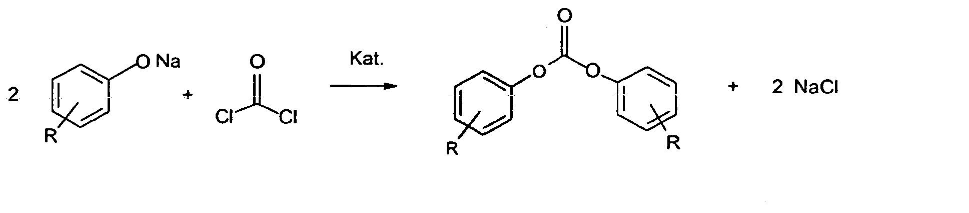

Die Herstellung von Diarylcarbonaten z.B. durch den Phasengrenzflächenprozess ist prinzipiell in der Literatur beschrieben, siehe z.B. in Chemistry and Physics of Polycarbonates, Polymer Reviews, H. Schnell, Vol. 9, John Wiley and Sons, Inc. (1964), S. 50/51.

In der Offenlegungsschrift US-A-4 016 190 wird ein Herstellungsverfahren von Diarylcarbonaten beschrieben, das bei Temperaturen oberhalb 65°C betrieben wird. Der pH-Wert wird bei diesem Verfahren zunächst niedrig (pH = 8 bis 9) und anschließend hoch (pH = 10 bis 11) eingestellt.

Optimierungen des Verfahrens durch Verbesserung der Durchmischung und Einhaltung eines engen Temperatur- und pH-Profils als auch Isolierung des Produkts werden in EP1219589 A1, EP1216981 A2, EP1216982 A2 und EP784048 A1 beschrieben.

Zur Herstellung von Diarylcarbonat nach dem Phasengrenzflächenverfahren erfolgt die Phosgenierung eines in wässrig-alkalischer Lösung oder Suspension vorgelegten Natriumsalzes eines Monophenols oder eines Gemisches verschiedener Monophenole in Gegenwart eines inerten organischen Lösungsmittels oder Lösungsmittelgemisches, welches neben der wässrigen eine zweite organische Phase ausbildet. Die entstehenden, hauptsächlich in der organischen Phase vorliegenden, Chlorameisensäurearylester werden mit Hilfe geeigneter Katalysatoren zu, in der organischen Phase gelösten, Diarylcarbonaten umgesetzt. Die organische Phase wird schließlich abgetrennt und das Diarylcarbonat durch verschiedene Aufarbeitungsschritte daraus isoliert.

Der erste Schritt zum Aufbau der Chlorameisensäurearylester und/oder Diarylcarbonate wird gemäß Stand der Technik beispielsweise in einem Umpumpreaktor durchgeführt; siehe z. B. EP 1 249 463 A1, US 2004/0158026 A1, US 6,613,868 B2. Im Umpumpreaktor erfolgen dabei das Mischen des eingebrachten Phosgens mit ebenfalls eingebrachtem Natriumsalz eines Monophenols (oder eines Gemisches verschiedener Monophenole) zu Chlorameisensäurearylestern und/oder Diarylcarbonaten. Ein gewisser Anteil des eingebrachten Monophenols befindet sich noch unreagiert im Gemisch. Zwar weist der Umpumpreaktor dahingehend Vorteile auf, dass Dosier- oder Konzentrationsschwankungen nicht ungedämpft bis zum Endprodukt durchschlagen können, sondern vielmehr durch Rückvermischung bei eingestelltem Umpumpverhältnis zu Zu- bzw. Ablauf der Reaktionsmischung eine Äquilibrierung von Schwankungen erreicht wird. Jedoch besteht ein signifikanter Nachteil darin, dass für großtechnisch vorteilhafte Parametereinstellungen noch erhebliche nicht vollständig umgesetzte Anteile Chlorameisensäurearylester in der Produktzusammensetzung vorhanden sind. Ein weiterer Nachteil ist darin zu sehen, dass Umpumpreaktoren relativ voluminöse Baugruppen darstellen.

Bei diesen bekannten Verfahren bereitet jedoch ein hoher Restphenolwert im Abwasser dieser Prozesse erhebliche Nachteile. Phenole können die Umwelt belasten und die Klärwerke vor eine erhöhte Abwasserproblematik stellen und machen aufwendige Reinigungsoperationen erforderlich.

So beschreibt WO 03/070639 A1 eine Entfernung der organischen Verunreinigungen im Abwasser durch eine Extraktion mit Methylenchlorid.

Üblicherweise wird die Natriumchlorid-haltige Lösung von Lösungsmitteln und organischen Resten befreit und muss dann entsorgt werden.

Bekannt ist aber auch, dass die Reinigung der Natriumchlorid-haltigen Abwässer gemäß der EP 1200359 B1 (WO2000078682 A1) oder US-A-6340736 durch Ozonolyse erfolgen kann und dann zum Einsatz bei der Natriumchlorid-Elektrolyse geeignet ist. Nachteil der Ozonolyse ist, dass dieses Verfahren sehr kostenintensiv ist.

Es bestand daher Bedarf nach einem kontinuierlichen Phasengrenzflächenverfahren zur Herstellung von Polycarbonat bzw. Diarylcarbonat, mit welchem die Belastung des aus diesem Verfahren resultierenden Abwassers mit Diphenol bzw. Monophenol weiter verringert werden kann. Weiterhin wünschenswert wäre dabei zudem, dass bei einem solchen Verfahren Polycarbonate mit enger Molekulargewichtsverteilung erhalten würden ohne dass z.B. Konzentrationsschwankungen ungedämpft bis zum Endprodukt durchschlagen können (kein plug-flow Verhalten). Auch bei der Herstellung von Diarylcarbonaten verhindert dieses Verfahren weitestgehend ungedämpfte Konzentrationsschwankungen der Chlorameisensäurearylester bis zum Endprodukt.

Überraschend wurde gefunden, dass eine signifikante Reduktion der Diphenol- bzw. Monophenol-Belastung des Abwassers erreicht werden kann, wenn im Phasengrenzflächenverfahren zur Herstellung von Polycarbonaten bzw. Diarylcarbonaten sowohl die Mischung der organischen und wässrigen Phase als auch der vorgelagerte Oligomerisierungsschritt bzw. die vorgelagerte Chlorameisensäurebildung in einer speziellen Pumpe erfolgt. Vorteilhafterweise schlagen auch bei diesem Verfahren Konzentrationsschwankungen nicht ungedämpft bis zum Endprodukt durch, so dass das erfindungsgemäße Verfahren die Vorteile des bekannten Verfahrens gemäß Stand der Technik beibehält, dessen Nachteile jedoch beseitigt. Eine erhöhte Diphenol- und/oder Monophenol-Belastung des Abwassers erfordert bekanntermaßen als Gegenmaßnahme eine Erhöhung des Phosgen- und Natronlaugezusatzes, um die Umsetzung des Diphenols bzw. Monophenols zu vervollständigen. Aufgrund der deutlich reduzierten Diphenol- und/oder Monophenol-Belastung des Abwassers gemäß erfindungsgemäßem Verfahren kann daher zudem eine solche Erhöhung des Phosgen- und Natronlaugezusatzes vermieden werden. Dies ist unter anderem aus ökonomischer Sicht von Vorteil. Weiterhin nimmt durch die effizientere Umsetzung bei der Herstellung von Polycarbonaten und Diarylcarbonaten die Chlorameisensäurearylestermenge ab, was eine geringere Aminkatalysatormenge erforderlich macht und somit zu einer Reduzierung der Urethannebenprodukte führt.

Gegenstand der vorliegenden Erfindung ist daher ein Verfahren zur kontinuierlichen Herstellung von Polycarbonaten oder Copolycarbonaten bzw. Diarylcarbonaten nach dem Phasengrenzflächenverfahren aus wenigstens einem Diphenol bzw. Monophenol, Phosgen und wenigstens einem Katalysator gegebenenfalls in Gegenwart wenigstens eines Kettenabbrechers und/oder Verzweigers, dadurch gekennzeichnet, dass
(a) kontinuierlich eine Mischung der organischen und wässrigen Phase erzeugt wird, wobei die organische Phase wenigstens ein für das Polycarbonat bzw. Diarylcarbonat geeignetes Lösungsmittel und ganz oder teilweise das Phosgen enthält und die wässrige Phase das oder die Diphenol(e) bzw. Monophenol(e), Wasser und Alkalilauge enthält,
(b) eine Umsetzung des oder der Diphenol(e) bzw. Monophenol(e) und Phosgen zu Oligocarbonaten bzw. zu Chlorameisensäurearylestern oder Diarylcarbonaten oder einer Mischung aus Chlorameisensäurearylestern und Diarylcarbonaten erfolgt,
(c) die Oligocarbonate bzw. Chlorameisensäurearylester und/oder Diarylcarbonate anschließend in wenigstens einem Reaktor unter Zugabe von weiterer Alkalilauge, gegebenenfalls Kettenabbrecher(n) und gegebenenfalls wenigstens eines weiteren Katalysators umgesetzt werden,
dadurch gekennzeichnet, dass die kontinuierliche Mischung der organischen und wässrigen Phase unter (a) und die Umsetzung zu Oligocarbonaten bzw. Chlorameisensäurearylester und/oder Diarylcarbonate unter (b) in einer oder mehreren Pumpe(n) erfolgen, die
- nach dem Stator-Rotor-Prinzip arbeitet (arbeiten)
- thermostatisierbar ist (sind) und
- wenigstens jeweils einen Zugang für die organische und die wässrige Phase und gegebenenfalls weitere Zugänge für Katalysator, Kettenabbrecher, Verzweiger und/oder zusätzlich Alkalilauge sowie wenigstens einen Ausgang für die oligocarbonathaltige bzw. Chlorameisensäurearylester-Diarylcarbonat Mischung aufweist (aufweisen).

Im erfindungsgemäßen Verfahren zur Herstellung von Polycarbonat bzw. Diarylcarbonat kann mindestens ein Teil der dabei anfallenden Alkalichlorid-haltigen Lösung in einer nachgeschalteten Alkalichlorid-Elektrolyse rückgeführt werden.

Die erfindungsgemäß verwendete(n) Pumpe(n) sind bevorzugt nach dem Ein- oder Mehrkammerprinzip aufgebaut.

Die organische Phase kann das erforderliche Phosgen vor der Mischung mit der wässrigen Phase bereits ganz oder teilweise enthalten. Bevorzugt enthält die organische Phase vor der Mischung bereits das gesamte erforderliche Phosgen inklusive des verwendeten Phosgenüberschusses.

Der Eintrag des Phosgens in die organische Phase bei der Herstellung von Polycarbonat kann gasförmig oder flüssig erfolgen. Der verwendete Überschuss an Phosgen, bezogen auf die Summe der eingesetzten Diphenole, liegt bevorzugt zwischen 3 und 100 Mol %, besonders bevorzugt zwischen 5 und 50 Mol %.

Der Eintrag des Phosgens in die organische Phase bei der Herstellung von Diarylcarbonat kann gasförmig oder flüssig erfolgen. Der verwendete Überschuss an Phosgen, bezogen auf die Summe der eingesetzten Monophenole, liegt bevorzugt zwischen 1 und 100 Mol %, besonders bevorzugt zwischen 2 und 50 Mol %.

Der pH-Wert der wässrigen Phase sollte über gegebenenfalls einmalige oder mehrfache Nachdosierung von Alkalilauge während und nach der Phosgendosierung im alkalischen Bereich bevorzugt zwischen 8,5 und 12 gehalten werden, während er nach der Katalysatorzugabe bei 10 bis 14 liegen sollte.

Die Phosgendosierung erfolgt vor der Mischung mit der wässrigen Phase ganz oder teilweise direkt in die organische Phase. Etwaige Teilmengen Phosgen können auch vor der Mischung mit der wässrigen Phase in die wässrige Phase oder aber nach der Mischung mit der wässrigen Phase in die resultierende Emulsion eindosiert werden. Weiterhin kann das Phosgen ganz oder teilweise in einen rückgeführten Teilstrom des Synthesegemisches aus beiden Phasen dosiert werden, wobei dieser Teilstrom vorzugsweise vor der Katalysatorzugabe rückgeführt wird. Besonders bevorzugt erfolgt die vollständige Phosgendosierung vor der Mischung mit der wässrigen Phase direkt in die organische Phase. In allen diesen Ausführungsformen sind die oben beschriebenen pH-Wertbereiche zu beachten und gegebenenfalls durch einmalige oder mehrfache Nachdosierung von Natronlauge oder Alkalilauge oder entsprechende Nachdosierung von Diphenol(at)lösung bzw. Monophenol(at)lösung einzuhalten. Ebenso muss der Temperaturbereich gegebenenfalls durch Kühlung oder Verdünnung eingehalten werden.

Die Synthese von Polycarbonaten aus Dihydroxydiarylalkanen (Diphenolen) bzw. von Diarylcarbonaten aus Monophenolen und Phosgen im alkalischen Milieu ist eine exotherme Reaktion und wird in einem Temperaturbereich von -5° C bis 100° C, bevorzugt 15°C bis 80°C, ganz besonders bevorzugt 25 bis 65°C durchgeführt.

Daher ist es erforderlich, dass die erfindungsgemäß für die Mischung und den vorgelagerten Oligomerisierungsschritt bzw. Chlorameisensäureesterbildung eingesetzte(n) Pumpe(n) auf eine Temperatur von -5°C bis 100°C, bevorzugt von 15°C bis 80°C, besonders bevorzugt von 25°C bis 65°C thermostatisierbar ist (sind).

Je nach Lösungsmittel oder Lösungsmittelgemisch kann unter Normaldruck oder unter Überdruck gearbeitet werden. Die erfindungsgemäß verwendete(n) Pumpe(n) können für Systemdrücke bis 350 bar und 450 °C ausgelegt werden. Sie sind somit für den Einsatz in einem breiten Verfahrensfenster geeignet.

Beim Phasengrenzflächenverfahren wird vorzugsweise in Druckbereichen von 1 bis 50 bar, besonders bevorzugt in Bereichen von 1 bis 10 bar gearbeitet.

Die organische Phase kann ein oder Mischungen mehrerer Lösungsmittel enthalten. Geeignete Lösungsmittel sind aromatische und/oder aliphatische chlorierte Kohlenwasserstoffe, bevorzugt Dichlormethan, Trichlorethylen, 1,1,1-Trichlorethan, 1,1,2-Trichlorethan und Chlorbenzol und deren Gemische. Es können jedoch auch aromatische Kohlenwasserstoffe wie Benzol, Toluol, m-/p-/o-Xylol oder aromatische Ether wie Anisol allein, im Gemisch oder zusätzlich oder im Gemisch mit chlorierten Kohlenwasserstoffen verwendet werden, bevorzugt sind Dichlormethan und Chlorbenzol und deren Gemische. Eine andere Ausführungsform der Synthese verwendet Lösungsmittel welche Polycarbonat nicht lösen sondern nur anquellen. Es können daher auch Nichtlösungsmittel für Polycarbonat in Kombination mit Lösungsmitteln verwendet werden. Wobei dann als Lösungsmittel auch in der wässrigen Phase lösliche Lösungsmittel wie Tetrahydrofuran, 1,3- oder 1,4-Dioxan oder 1,3-Dioxolan verwendet werden können, wenn der Lösungsmittelpartner die zweite organische Phase bildet.

Geeignete Diphenole sind solche der allgemeinen Formel,

HO-Z-OH

worin Z ein divalenter organischer Rest mit 6 bis 30 Kohlenstoffatomen ist, der eine oder mehrere aromatische Gruppen enthält. Beispiele solcher Verbindungen, die in das erfindungsgemäße Verfahren eingesetzt werden können sind Dihydroxydiarylalkane wie Hydrochinon, Resorcin, Dihydroxydiphenyl, Bis-(hydroxyphenyl)-alkane, Bis(hydroxy-phenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α,α'-Bis-(hydroxyphenyl)-diisopropylbenzole, Bis-(hydroxyphenyl)phtalimidine sowie deren alkylierte, kernalkylierte und kernhalogenierte Verbindungen.

Bevorzugte Diphenole sind 4,4'-Dihydroxydiphenyl, 2,2-Bis-(4-hydroxyphenyl)-1-phenyl-propan, 1,1-Bis-(4-hydroxyphenyl)-phenyl-ethan, 2,2-Bis-(4-hydroxyphenyl)propan (Bisphenol A (BPA)), 2,4-Bis-(4-hydroxyphenyl)-2-methylbutan, 1,3-Bis-[2-(4-hydroxyphenyl)-2-propyl]benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol, 1,1-Bis-(4-hydroxyphenyl)-cyclohexan, 2-Hydrocarbyl-3,3-bis(4-hydroxyaryl)phthalimidine, 3,3-Bis(4-hydroxyaryl)-1-aryl-1H-indol-2-on, 2,2-Bis-(4-hydroxyaryl)-1-aryl-1H-indol-2-on und 1,1-Bis-(4-hydroxyphenyl)-3,3,5-trimethylcyclohexan (Bisphenol TMC).

Besonders bevorzugte Diphenole sind 4,4'-Dihydroxydiphenyl, 1,1-Bis-(4-hydroxyphenyl)-phenylethan, 2,2-Bis-(4-hydroxyphenyl)-propan (Bisphenol A (BPA)), 2,2-Bis(3,5-dimethyl-4-hydroxyphenyl)-propan, 1, 1 -Bis-(4-hydroxyphenyl)-cyclohexan, 2-Hydrocarbyl-3,3-bis(4-hydroxyphenyl)phthalimidin, 3,3-Bis(4-hydroxyphenyl)-1-phenyl-1H-indol-2-on, 2,2-Bis(4-hydroxyphenyl)-1-phenyl-1H-indol-2-on, 3,3-Bis(4-hydroxyphenyl)-1-methyl-1H-indol-2-on, 2,2-Bis(4-hydroxyphenyl)-1-methyl-1H-indol-2-on, 3,3-Bis(4-hydroxyphenyl)-N-methyl-phthalimidin, 3,3-Bis(4-hydroxyphenyl)-N-phenyl-phthalimidin und 1,1-Bis-(4-hydroxyphenyl)-3,3,5-trimethylcyclohexan (Bisphenol TMC).

Diese und weitere geeignete Diphenole sind z.B. in den US-A 2 999 835, US-A 3,148,172 US-A 2,991,273, US-A 3 271 367, US-A 4,982,014 und US-A 2,999,846 in den deutschen Offenlegungsschriften DE-A 1 570 703, DE-A 2 063 050, DE-A 2 036 052, DE-A 2 211 956 und DE-A 3 832 396, der französischen Patentschrift FR-A 1 561 518, in der Monographie von H. Schnell, Chemistry and Physics of Polycarbonates, Interscience Publishers, New York 1964, S. 28ff; S.102ff und von D.G. Legrand, J.T. Bendler, Handbook of Polycarbonate Science and Technology, Marcel Dekker New York 2000, S. 72ff. beschrieben.

Im Falle der erfindungsgemäßen Herstellung von Homopolycarbonaten wird nur ein Diphenol eingesetzt, im Falle der erfindungsgemäßen Herstellung von Copolycarbonaten werden mehrere Diphenole eingesetzt, wobei selbstverständlich die verwendeten Diphenole, wie auch alle anderen der Synthese zugesetzten Chemikalien und Hilfsstoffe mit den aus ihrer eigenen Synthese, Handhabung und Lagerung stammenden Verunreinigungen kontaminiert sein können, obwohl es wünschenswert ist, mit möglichst sauberen Rohstoffen zu arbeiten.

Besonders geeignete Monophenole zum Einsatz in dem neuen Verfahren sind Phenole der Formel (I) 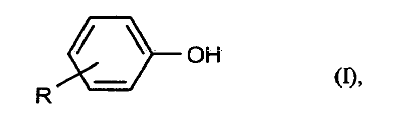 worin
- R: Wasserstoff, Halogen oder ein verzweigter oder unverzweigter C₁- bis C₉-Alkylrest oder Alkoxycarbonylrest ist.

Bevorzugt sind also Phenol, Alkylphenole wie Kresole, p-tert.-Butylphenol, p-Cumyl-phenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol und 2,4,6-Tribromphenol oder Salicylsäuremethylester. Besonders bevorzugt ist Phenol.

Unter Alkalilauge ist im Rahmen der Erfindung bevorzugt Natronlauge, Kalilauge oder Mischungen aus diesen, besonders bevorzugt Natronlauge zu verstehen.

Bei der Herstellung von Diarylcarbonaten kann das eingesetzte Alkali zur Bildung des Phenolats eine Alkalilauge mit Hydroxiden aus der Reihe: Na-, K-, Li-Hydroxid sein. Bevorzugt ist Natronlauge, und wird im neuen Verfahren vorzugsweise als 10 bis 55 Gew.-%-ige Lösung eingesetzt. Das eingesetzte Alkalihydroxid bzw. die Alkalilauge kann im Falle von Natriumhydroxid bzw. Natronlauge beispielsweise nach dem Amalgamverfahren oder dem sogenannten Membranverfahren hergestellt worden sein. Beide Verfahren werden seit langer Zeit benutzt und sind dem Fachmann geläufig. Bevorzugt wird im Falle von Natronlauge solche hergestellt nach dem Membranverfahren verwendet.

Die wässrige Phase im erfindungsgemäßen Verfahren zur Herstellung von Polycarbonaten enthält Alkalilauge, ein oder mehrere Diphenole und Wasser, wobei die Konzentration dieser wässrigen Lösung bezüglich der Summe der Diphenole, nicht als Natriumsalz sondern als freies Diphenol gerechnet, vorzugsweise zwischen 1 und 30 Gew. %, besonders bevorzugt zwischen 3 und 25 Gew. %, bezogen auf das Gesamtgewicht der wässrigen Lösung, variieren kann. Ganz besonders bevorzugt kann die Konzentration dieser wässrigen Lösung bezüglich der Summe der Diphenole zwischen 3 und 8 Gew. % für Polycarbonate mit einem M_{w} von größer als 45000 gmol⁻¹ und zwischen 12 bis 22 Gew. % für Polycarbonate mit einem M_{w} von 45000 oder kleiner variieren. Dabei kann es bei höheren Konzentrationen notwendig sein, die Lösungen zu temperieren. Die zur Lösung der Diphenole verwendete Alkalihydroxid, z.B. Natrium- oder Kaliumhydroxid, kann fest oder als entsprechende wässrige Alkalilauge verwendet werden. Die Konzentration der Alkalilauge richtet sich nach der Zielkonzentration der angestrebten Diphenollösung, liegt aber in der Regel zwischen 5 und 25 Gew.-%, bevorzugt 5 und 10 Gew.-% bezogen auf 100 %ige Alkalilauge, oder aber wird konzentrierter gewählt und anschließend mit Wasser verdünnt. Bei dem Verfahren mit anschließender Verdünnung werden Alkalilaugen mit Konzentrationen zwischen 15 und 75 Gew.-%, bevorzugt 25 und 55 Gew.-%, gegebenenfalls temperiert, verwendet. Der Alkaligehalt pro mol Diphenol ist von der Struktur des Diphenols abhängig, bewegt sich aber in der Regel von 0,25 mol Alkali / mol Diphenol bis 5,00 mol Alkali / mol Diphenol, bevorzugt von 1,5 bis 2,5 Mol Alkali / mol Diphenol und im besonders bevorzugten Fall, dass Bisphenol A als alleiniges Diphenol verwendet wird, von 1,85 bis 2,15 mol Alkali. Wird mehr als ein Diphenol verwendet, so können diese zusammen gelöst werden. Da die Löslichkeit von Diphenolen sehr stark von der verwendeten Alkalimenge abhängt, kann es jedoch vorteilhaft sein, statt einer Lösung mit zwei Diphenolen besser zwei Lösungen mit je einem in einer geeigneten Alkalilauge gelösten Diphenol zu haben, die dann getrennt so dosiert werden, dass das richtige Mischungsverhältnis entsteht. Weiterhin kann es von Vorteil sein, das oder die Diphenol(e) nicht in Alkalilauge sondern in mit zusätzlichem Alkali ausgestatteter, verdünnter Diphenollösung zu lösen. Die Lösevorgänge können von festem Diphenol, meist in Schuppen oder Prillform oder auch von geschmolzenem Diphenol ausgehen. Das eingesetzte Alkalihydroxid bzw. die Alkalilauge kann im Falle von Natriumhydroxid bzw. Natronlauge beispielsweise nach dem Amalgamverfahren oder dem sogenannten Membranverfahren hergestellt worden sein. Beide Verfahren werden seit langer Zeit benutzt und sind dem Fachmann geläufig. Bevorzugt wird im Falle von Natronlauge solche hergestellt nach dem Membranverfahren verwendet.

In einer solchen wässrigen Lösung und/oder der wässrigen Phase liegen das oder die Diphenol(e) ganz oder teilweise in Form der entsprechenden Alkalisalze bzw. Dialkalisalze vor.

Die Reaktion b) kann bei der Herstellung von Diarylcarbonaten durch Katalysatoren, wie tertiäre Amine, N-Alkylpiperidine oder Oniumsalze beschleunigt werden. Bevorzugt werden Tributylamin, Triethylamin und N-Ethylpiperidin verwendet.

Der hier eingesetzte Amin-Katalysator kann offenkettig oder zyklisch sein, besonders bevorzugt ist Triethylamin und Ethylpiperidin. Der Katalysator wird im neuen Verfahren vorzugsweise als 1 bis 55 Gew.-%-ige Lösung eingesetzt.

Unter Oniumsalzen werden hier Verbindungen wie NR₄X verstanden, wobei R ein Alkyl und/oder Arylrest und/oder ein H sein kann und X ein Anion ist.

Phosgen kann in dem Verfahrensschritt b) flüssig, gasförmig oder gelöst in einem inerten Lösungsmittel eingesetzt werden.

In dem neuen Verfahren in Schritt b) bevorzugt verwendbare inerte organische Lösungsmittel sind beispielsweise Dichlormethan, Toluol, die verschiedenen Dichlorethane und Chlorpropanverbindungen, Chlorbenzol und Chlortoluol. Vorzugsweise wird Dichlormethan eingesetzt.

Die Reaktionsführung für Schritt b) erfolgt bevorzugt kontinuierlich und besonders bevorzugt in einer Pfropfenströmung mit geringer Rückvermischung. Dies kann somit beispielsweise in Rohrreaktoren geschehen. Die Durchmischung der zwei Phasen (wässrige und organische Phase) kann durch eingebaute Rohrblenden, statische Mischer und/oder beispielsweise Pumpen realisiert werden.

Die Reaktion gemäß Schritt b) erfolgt bei der Herstellung von Diarylcarbonaten besonders bevorzugt zweistufig.

In der ersten Stufe des bevorzugten Verfahrens wird die Reaktion durch Zusammenbringen der Edukte Phosgen, des inerten Lösungsmittels, welches bevorzugt erst als Lösungsmittel für das Phosgen dient, und des Monophenols, welches vorzugsweise zuvor bereits in der Alkalilauge gelöst ist, gestartet. Die Verweilzeit liegt typischerweise in der ersten Stufe im Bereich von 2 Sekunden bis 300 Sekunden, besonders bevorzugt im Bereich von 4 Sekunden bis 200 Sekunden. Der pH-Wert der ersten Stufe wird durch das Verhältnis von Alkalilauge/ Monophenol /Phosgen bevorzugt so eingestellt, dass der pH-Wert im Bereich von 11,0 bis 12,0, bevorzugt 11,2 bis 11,8, besonders bevorzugt bei 11,4 bis 11,6 liegt. Die Reaktionstemperatur der ersten Stufe wird durch Kühlung bevorzugt unterhalb von 40°C, besonders bevorzugt unterhalb von 35°C gehalten.

In der zweiten Stufe des bevorzugten Verfahrens wird die Reaktion zum Diarylcarbonat komplettiert. Die Verweilzeit beträgt beim bevorzugten Prozess 1 Minute bis 2 Stunden, bevorzugt 2 Minuten bis 1 Stunde, ganz besonders bevorzugt 3 Minuten bis 30 Minuten. In der zweiten Stufe des bevorzugten Verfahrens wird durch permanente Kontrolle des pH-Werts (wird im kontinuierlichen Verfahren bevorzugt Online nach grundsätzlich bekannten Verfahren gemessen) und entsprechende Einstellung des pH-Wertes durch Zugabe der Alkalilauge geregelt. Die Menge zugeführten Alkalilauge wird insbesondere so eingestellt, dass der pH-Wert der Reaktionsmischung in der zweiten Verfahrensstufe im Bereich von 7,5 bis 10,5, bevorzugt 8 bis 9,5, ganz besonders bevorzugt 8,2 bis 9,3 liegt. Die Reaktionstemperatur der zweiten Stufe wird durch Kühlung bei bevorzugt unterhalb von 50°C, besonders bevorzugt unterhalb von 40°C, ganz besonders bevorzugt unterhalb von 35°C gehalten.

Die in dieser Anmeldung aufgeführten allgemeinen oder in Vorzugsbereichen aufgeführten Parameter bzw. Erläuterungen können jedoch auch untereinander, also zwischen den jeweiligen Bereichen und Vorzugsbereichen beliebig kombiniert werden.

Im bevorzugten Verfahren zur Herstellung von Diarylcarbonaten wird in Schritt b) Phosgen in Bezug auf das Monophenol im Mol-Verhältnis von 1 : 2 bis 1 : 2,2 eingesetzt. Das Lösungsmittel wird so zugemischt, dass das Diarylcarbonat in einer 5 bis 60 %igen Lösung, bevorzugt 20 bis 45 %igen Lösung nach der Reaktion vorliegt.

Als Katalysatoren für das erfindungsgemäße Verfahren zur Herstellung von Diarylcarbonaten eignen sich bevorzugt tertiäre Amine, wie z.B. Triethylamin, Tributylamin, Trioctylamin, N-Ethylpiperidin, N-Methylpiperidin oder N-i/n-Propylpiperidin, quartäre Ammoniumsalze wie z.B. Tetrabutylammonium-, Tributylbenzyl-ammonium-, Tetraethylammoniumhydroxid, -chlorid, - bromid, -hydrogensulfat oder -tetrafluoro-borat sowie die den vorangehend genannten Ammoniumverbindungen entsprechenden Phosphoniumverbindungen. Diese Verbindungen sind als typische Phasengrenzflächenkatalysatoren in der Literatur beschrieben, kommerziell erhältlich und dem Fachmann geläufig. Die Katalysatoren können einzeln, im Gemisch oder auch neben- und nacheinander dem erfindungsgemäßen Verfah-ren zugesetzt werden, gegebenenfalls auch vor der Phosgenierung, bevorzugt sind jedoch Dosierungen nach der Phosgeneintragung, es sei denn, es wird eine Oniumverbindung - d.h. Ammonium- oder Phosphoniumverbindung - oder Gemische aus Oniumverbindungen als Katalysatoren verwendet. Im Falle einer solchen Oniumsalzkatalyse ist eine Zugabe vor der Phosgendosierung bevorzugt. Die Dosierung des Katalysators oder der Katalysatoren kann in Substanz, in einem inerten Lösungsmittel, vorzugsweise dem oder einem derer der organischen Phase bei der Diarylcarbonatsynthese, oder auch als wässrige Lösung erfolgen. Im Falle der Verwendung von tertiären Aminen als Katalysator kann beispielsweise deren Dosierung in wässriger Lösung als deren Ammoniumsalze mit Säuren, bevorzugt Mineralsäuren, insbesondere Salzsäure, erfolgen. Bei Verwendung mehrerer Katalysatoren oder der Dosierung von Teilmengen der Katalysatorgesamtmenge können auch unterschiedliche Dosierungsweisen an verschiedenen Orten oder zu verschiedenen Zeiten vorgenommen werden. Die Gesamtmenge der verwendeten Katalysatoren liegt zwischen 0,0001 und 1,0 Mol-%, bevorzugt zwischen 0,001 und 0,2 Mol-%, bezogen auf Mole eingesetzte Monophenole.

Zur Bildung des Mono-Phenolats wird bevorzugt 1 mol Alkali / mol Monophenol bis 5,00 mol Alkali / mol Monophenol, besonders bevorzugt von 1,1 bis 2,5 Mol Alkali / mol Monophenol eingesetzt. Die Lösevorgänge können von festem Phenol oder auch von geschmolzenem Phenol ausgehen. Das eingesetzte Alkalihydroxid bzw. die Alkalilauge kann im Falle von Natriumhydroxid bzw. Natronlauge beispielsweise nach dem Amalgamverfahren oder dem sogenannten Membranverfahren hergestellt worden sein. Beide Verfahren werden seit langer Zeit benutzt und sind dem Fachmann geläufig. Bevorzugt wird im Falle von Natronlauge solche hergestellt nach dem Membranverfahren verwendet.

In einer solchen wässrigen Lösung und/oder der wässrigen Phase liegen das oder die Monophenol(e) ganz oder teilweise in Form der entsprechenden Alkalisalze vor.

Im erfindungsgemäßen Verfahren werden für die Mischung der organischen und wässrigen Phase und den vorgelagerten Oligomerisierungsschritt bzw. Chlorameisensäurearylesterherstellungsschritt eine oder mehrere Pumpe(n) verwendet. Mehrere Pumpen können insbesondere im industriellen Maßstab aus Kapazitätsgründen verwendet werden. Im Falle des Einsatzes mehrerer Pumpen können diese sowohl parallel als auch in Reihe geschaltet sein. Bevorzugt wäre eine Parallelschaltung mehrerer Pumpen. Die Pumpe übernimmt im erfindungsgemäßen Verfahren die Funktion eines Mischaggregats zum Vermischen der organischen und wässrigen Phase zur Herstellung einer entsprechenden Emulsion, die Funktion des Reaktionsraumes für den Aufbau der Oligocarbonate sowie die Funktion der kontinuierlichen Förderung des Reaktionsgutes.

Die erfindungsgemäß verwendeten Pumpen arbeiten nach dem Stator-Rotor-Prinzip. Hierdurch wird bei der Mischung der beiden Phasen insbesondere Energie über Scherung in das Reaktionsgemisch eingetragen. Dadurch weist die resultierende Emulsion eine ausreichend große Phasengrenzfläche auf, so dass eine optimale Reaktion an dieser Phasengrenzfläche erfolgen kann.

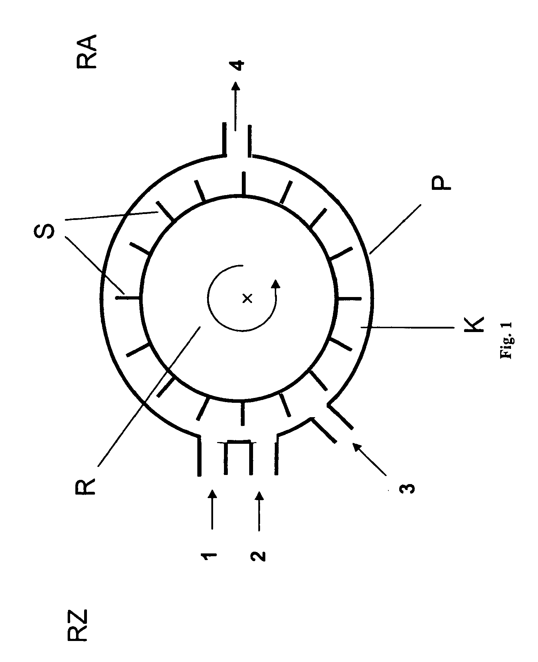

Bei den erfindungsgemäß verwendeten Pumpen stellt das Pumpengehäuse vorzugsweise den Stator und im Inneren der Pumpe befinden sich ein oder mehrere Rotoren. Im Falle mehrerer Rotoren sind diese vorzugsweise auf der gleichen Rotorachse aufgebracht. Bei den erfindungsgemäß verwendeten Pumpen handelt es sich vorzugsweise um Kreiselpumpen. In bevorzugten Ausführungsformen sind dies solche Kreiselpumpen, deren Laufrad (Rotor) eine peripherale Bauform aufweist (Peripheralradpumpe). In weiteren bevorzugten Ausführungsformen sind dies solche Kreiselpumpen, deren Laufrad (Rotor) eine radiale Bauform aufweist (Radialradpumpe). Erfindungsgemäß besonders bevorzugt sind Pumpen, die nach dem Prinzip der Peripheralradpumpe arbeiten. Solche Kreiselpumpen und deren Funktionsweise sind dem Fachmann bekannt (vgl. z.B. DE 42 20 239 A1) und kommerziell erhältlich. Das Prinzip einer solchen erfindungsgemäß verwendeten Pumpe ist beispielhaft und schematisch in Fig. 1 gezeigt. Es sind sowohl Ausführungsformen mit einfachen oder Mehrfachspalttöpfen, wie z.B. Doppelspalttöpfen, geeignet.

Fig. 1: Schematische Darstellung des Prinzips einer Peripheralradpumpe mit rotierender Innenvorrichtung

Die in Fig. 1 verwendeten Abkürzungen stehen für
- 1: Phosgen / Lösungsmittel-Zustrom
- 2: Zustrom wässrig/alkalische Natriumbisphenolat-Lösung bzw. Natriummonophenolat- Lösung
- 3: Natronlauge-Zustrom (NaOH-Zustrom)
- 4: Abgeführtes Reaktionsgemisch
- R: Rotierende Innenvorrichtung
- S: Schaufeln an der rotierenden Innevorrichtung
- K: Umlaufkanal
- RZ: Reaktionszulaufseite (Saugseite)
- RA: Reaktionsablaufseite (Druckseite)
- P: Statisches Gehäuse der Peripheralradpumpe mit rotierender Innenvorrichtung

Die in Fig. 1 dargestellte Peripheralradpumpe weist ein statisches Gehäuse P, eine rotierende Innenvorrichtung R mit Schaufeln S (hier als Schaufelrad ausgebildet) und einen daraus resultierenden im Gehäuse umlaufenden Kanal K von der Saug- bis zur Druckseite hin auf. Die Peripheralpumpe kann für eine Strömungspumpe relativ hohe Drücke bei kleinen Fördermengen und kleiner Bauform schaffen. Während der Förderung gibt das transportierte Reaktionsgemisch zwischen den Schaufeln S seine Energie an das im Umlaufkanal geförderte Reaktionsgemisch ab. Die Druckerhöhung erfolgt durch Impulsaustausch, indem die mit größerer Geschwindigkeit strömende Flüssigkeit in den Schaufeln des Laufrades die Energie an die mit geringerer Geschwindigkeit rotierende Flüssigkeit im Umlaufkanal abgibt.

Die erfindungsgemäß verwendeten Pumpen sind thermostatisierbar, d.h. weisen einen Ein- und Auslass für eine Thermostatisierflüssigkeit auf. Durchflussraum für diese Thermostatisierflüssigkeit und Reaktionsraum für das Reaktionsgut sind dabei derart voneinander getrennt, dass kein Stoffaustausch aber ein Wärmeaustausch erfolgen kann.

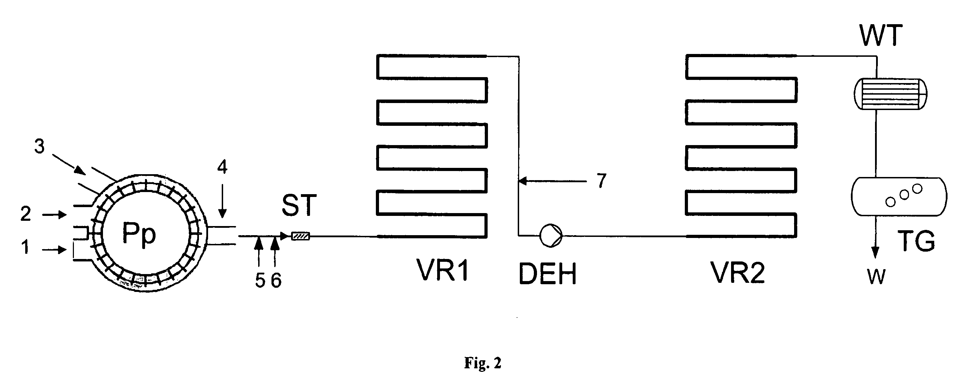

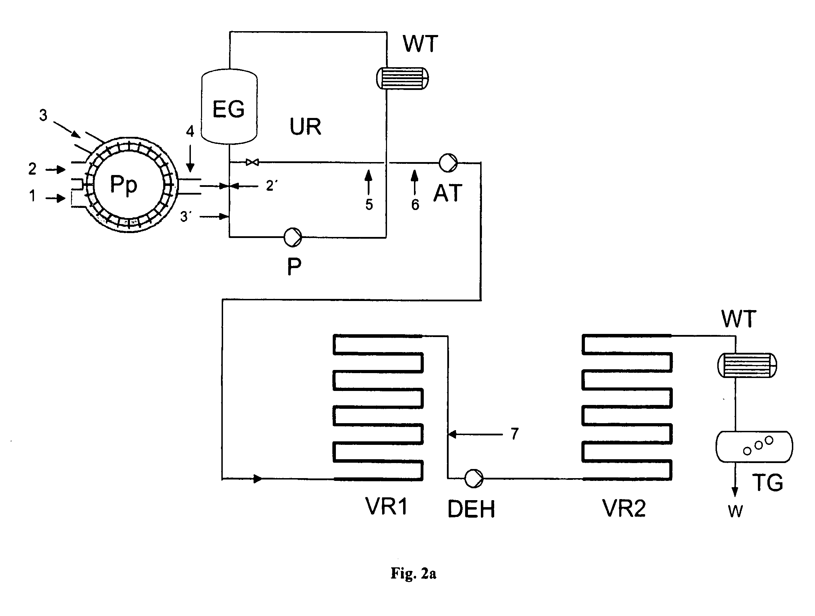

Die erfindungsgemäß verwendeten Pumpen weisen als Zuführung zum Reaktionsraum wenigstens jeweils einen Zugang für die organische und die wässrige Phase sowie wenigstens einen Ausgang für die oligocarbonathaltige Reaktionsmischung (bei der Polycarbonatherstellung), bzw. für die in der 1. Stufe gebildete Reaktionsmischung bei der Diarylcarbonatherstellung, auf (vgl. Fig. 1). In bevorzugten Ausführungsformen sind weitere Zugänge für zusätzliche Alkalilauge und/oder Diphenol(at)lösung bzw. Monophenol(at)lösung und gegebenenfalls für Kettenabbrecher und Verzweiger bevorzugt. Der Katalysator wird bevorzugt zu einem späteren Zeitpunkt einem nachgeschalteten Verweilreaktor zugegeben. Ein schematischer beispielhafter Aufbau für eine solche erfindungsgemäße Reaktionsführung ist in Fig. 2 dargestellt. Die Darstellung in Fig. 2 ist hinsichtlich der Anzahl der nachgeschalteten Verweilreaktoren lediglich als beispielhaft zu betrachten. Es können auch mehr oder weniger als zwei Verweilreaktoren nachgeschaltet werden.

Fig. 2: Schematischer Aufbau für die Herstellung von Polycarbonat bzw. Diarylcarbonat nach dem Phasengrenzflächenverfahren unter Verwendung einer Peripheralradpumpe

Die in Fig. 2 verwendeten Abkürzungen stehen für
- 1: Phosgen / Lösungsmittel-Zustrom
- 2: Zustrom wässrig/alkalische Natriumbisphenolat-Lösung bzw. Natriummonophenolat- Lösung
- 3: Erster Natronlauge-Zustrom (NaOH-Zustrom 1)
- 4: Abgeführtes Reaktionsgemisch
- 5: Zweiter Natronlauge-Zustrom (NaOH-Zustrom 2)
- 6: Kettenabbrecher bei der Herstellung von Polcarbonat bzw. Katalysator bei der Herstellung von Diarylcarbonat (optional)
- 7: Katalysator
- Pp: Peripheralradpumpe
- ST: Statikmischer
- VR1: Verweilreaktor 1
- VR2: Verweilreaktor 2 (optional)
- WT: Wärmetauscher
- DEH: Druckerhöhungspumpe
- TG: Trenngefäß
- W: nachträgliche Wäsche

In einer weiteren Ausführungsform des erfindungsgemäßen Verfahrens kann der erfindungsgemäß verwendeten Pumpe auch ein Umpumpreaktor anstelle eines Verweilreaktors nachgeschaltet sein. Diese Ausführungsvariante des erfindungsgemäßen Verfahrens hätte den Vorteil, dass bestehende Anlagen durch Vorschalten einer erfindungsgemäß verwendeten Pumpe genutzt werden könnten, ohne dass weitreichende Umbauten an den Anlagen erforderlich wären. Ein schematischer beispielhafter Aufbau für eine solche erfindungsgemäße Reaktionsführung ist in Fig. 2a dargestellt. Die Darstellung in Fig. 2a ist hinsichtlich der Anzahl der nachgeschalteten Verweilreaktoren lediglich als beispielhaft zu betrachten. Es können auch mehr oder weniger als zwei Verweilreaktoren nachgeschaltet werden.

In einer Verfahrensvariante mit der erfindungsgemäß verwendeten Pumpe nachgeschaltetem Umpumpreaktor können die Edukte, wie z.B. die Diphenolatlösung bzw. Monophenolatlösung oder der erste Natronlauge-Zustrom, entweder vollständig der Pumpe zugegeben oder gegebenenfalls anteilig erst dem nachgeschalteten Umpumpreaktor zugeführt werden.

Fig. 2a: Schematischer Aufbau für die Herstellung von Polycarbonat bzw. Diarylcarbonat nach dem Phasengrenzflächenverfahren unter Verwendung einer Peripheralradpumpe mit nachgeschaltetem Umpumpreaktor

Die in Fig. 2a zusätzlich zu den in Fig. 2 verwendeten Abkürzungen stehen für

2' optionaler Zustrom eines Anteils wässrig/alkalischer Natriumbisphenolat-Lösung bzw. Natriummonophenolat-Lösung
- 3': optionaler Zustrom eines Anteils des ersten Natronlauge-Zustrom (NaOH-Zustrom 1')
- EG: Entgasungsgefäß
- UR: Umpumpreaktor
- AT: Austragspumpe
- P: Pumpe zur Aufrechterhaltung des Umlaufs

Die Verweilzeit der Reaktionsmischung in der erfindungsgemäß verwendeten Pumpe beträgt bevorzugt 20 Sekunden bis 10 Minuten, besonders bevorzugt 30 Sekunden bis 5 Minuten und ganz besonders bevorzugt 30 Sekunden bis 3 Minuten.

Zur Regelung des Molekulargewichtes des Polycarbonats kann gegebenenfalls der Zusatz von einem oder mehreren monofunktionellen Kettenabbrecher(n), wie Phenol oder Alkylphenolen, insbesondere Phenol, p-tert.Butylphenol, iso-Octylphenol, Cumylphenol, deren Chlorkohlensäureester oder Säurechloride von Monocarbonsäuren bzw. Gemischen aus diesen Kettenabbrechern erforderlich sein. Solche Kettenabbrecher werden gegebenenfalls entweder mit dem oder den Dihydroxydiarylalkan(en) der Reaktion zugeführt oder aber zu jedem beliebigen Zeitpunkt der Synthese zugesetzt, solange im Reaktionsgemisch noch Phosgen oder Chlorkohlensäureendgruppen vorhanden sind bzw. im Falle der Säurechloride und Chlorkohlensäureester als Kettenabbrecher solange genügend phenolische Endgruppen des sich bildenden Polymers zur Verfügung stehen. Vorzugsweise werden der oder die Kettenabbrecher jedoch nach der Phosgenierung an einem Ort oder zu einem Zeitpunkt zugegeben, an oder zu dem kein Phosgen mehr vorliegt, aber der Katalysator noch nicht zudosiert wurde, d.h. sie können vor dem Katalysator, mit dem Katalysator zusammen oder parallel dazu zudosiert werden.

In der gleichen Weise können bei der Herstellung von Polycarbonaten gegebenenfalls ein oder mehrere Verzweiger oder Verzweigermischungen der Synthese zugesetzt werden. Üblicherweise werden solche Verzweiger jedoch vor dem oder den Kettenabbrecher(n) zugesetzt. Beispielsweise werden als Verzweiger Trisphenole, Quarterphenole, Säurechloride von Tri- oder Tetracarbonsäuren oder auch Gemische der Polyphenole oder der Säurechloride verwendet.

Beispiele für als Verzweiger geeignete Verbindungen mit drei oder mehr als drei phenolischen Hydroxylgruppen sind Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis-(4,4-bis-(4-hydroxyphenyl)-cyclohexyl]-propan, 2,4-Bis-(4-hydroxyphenyl-isopropyl)-phenol, Tetra-(4-hydroxyphenyl)-methan.

Beispiele für sonstige als Verzweiger geeignete trifunktionelle Verbindungen sind 2,4-Dihydroxybenzoesäure, Trimesinsäure, Cyanurchlorid und 3,3-Bis-(3-methyl-4-hydroxyphenyl)-2-oxo-2,3-dihydroindol.

Besonders bevorzugte Verzweiger sind 3,3-Bis-(3-methyl-4-hydroxyphenyl)-2-oxo-2,3-dihydroindol und 1,1,1-Tri-(4-hydroxyphenyl)-ethan.

Als Katalysatoren für das erfindungsgemäße Verfahren zur Herstellung von Polycarbonaten eignen sich bevorzugt tertiäre Amine, wie z.B. Triethylamin, Tributylamin, Trioctylamin, N-Ethylpiperidin, N-Methylpiperidin oder N-i/n-Propylpiperidin, quartäre Ammoniumsalze wie z.B. Tetrabutylammonium-, Tributylbenzylammonium-, Tetraethylammoniumhydroxid, -chlorid, - bromid, -hydrogensulfat oder -tetrafluoroborat sowie die den vorangehend genannten Ammoniumverbindungen entsprechenden Phosphoniumverbindungen. Diese Verbindungen sind als typische Phasengrenzflächenkatalysatoren in der Literatur beschrieben, kommerziell erhältlich und dem Fachmann geläufig. Die Katalysatoren können einzeln, im Gemisch oder auch neben- und nacheinander dem erfindungsgemäßen Verfahren zugesetzt werden, gegebenenfalls auch vor der Phosgenierung, bevorzugt sind jedoch Dosierungen nach der Phosgeneintragung, es sei denn, es wird eine Oniumverbindung - d.h. Ammonium- oder Phosphoniumverbindung - oder Gemische aus Oniumverbindungen als Katalysatoren verwendet. Im Falle einer solchen Oniumsalzkatalyse ist eine Zugabe vor der Phosgendosierung bevorzugt. Die Dosierung des Katalysators oder der Katalysatoren kann in Substanz, in einem inerten Lösungsmittel, vorzugsweise dem oder einem derer der organischen Phase bei der Polycarbonatsynthese, oder auch als wässrige Lösung erfolgen. Im Falle der Verwendung von tertiären Aminen als Katalysator kann beispielsweise deren Dosierung in wässriger Lösung als deren Ammoniumsalze mit Säuren, bevorzugt Mineralsäuren, insbesondere Salzsäure, erfolgen. Bei Verwendung mehrerer Katalysatoren oder der Dosierung von Teilmengen der Katalysatorgesamtmenge können natürlich auch unterschiedliche Dosierungsweisen an verschiedenen Orten oder zu verschiedenen Zeiten vorgenommen werden. Die Gesamtmenge der verwendeten Katalysatoren liegt zwischen 0,001 bis 10 Mol %, bevorzugt 0,01 bis 8 Mol %, besonders bevorzugt 0,05 bis 5 Mol % bezogen auf Mole eingesetzte Diphenole.

Oligocarbonate sind im Rahmen der Erfindung bevorzugt solche der allgemeinen Formeln (I), (II) und/oder (III), 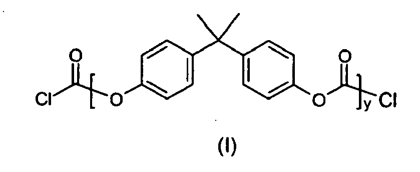 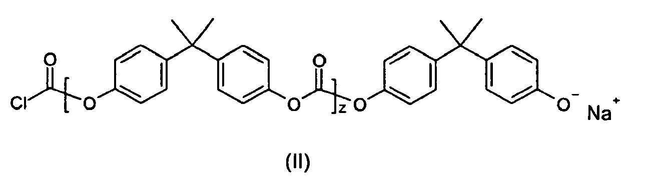 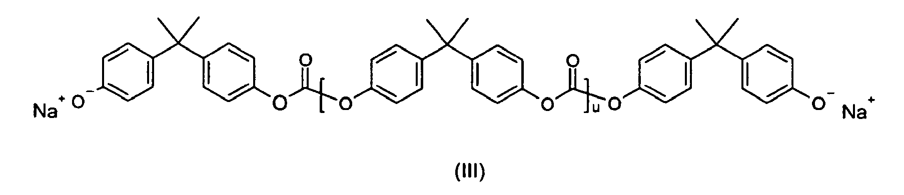 worin
- u: für 0 oder eine ganze Zahl von 1 bis 20, bevorzugt für 0 oder eine ganze Zahl von 1 bis 12, besonders bevorzugt für 0 oder eine ganze Zahl von 1 bis 8 steht,
- y: für eine ganze Zahl von 1 bis 20, bevorzugt von 1 bis 12, besonders bevorzugt von 1 bis 8, und
- z: für 0 oder eine ganze Zahl von 1 bis 20, bevorzugt für 0 oder eine ganze Zahl von 1 bis 12, besonders bevorzugt für 0 oder eine ganze Zahl von 1 bis 8 steht.

Solche Oligomere weisen bevorzugt ein mittleres Molekulargewicht M_{w} von bis zu 5000 g/mol, bevorzugt bis zu 3500 g/mol, ganz besonders bevorzugt bis zu 2000 g/mol auf.

Bei den im Rahmen dieser Anmeldung angegebenen mittleren Molekulargewichtsmittel handelt es sich um Gewichtsmittel (M_{w}), welche durch Gelpermeationschromotographie (GPC) unter Nutzung von Polycarbonat als Standard ermittelt wurden. Die Detektion der Eluatsignale kann beispielsweise durch Nutzung des Brechungsindex oder durch UV-Absorption im Bereich von z.B. 254 nm erfolgen.

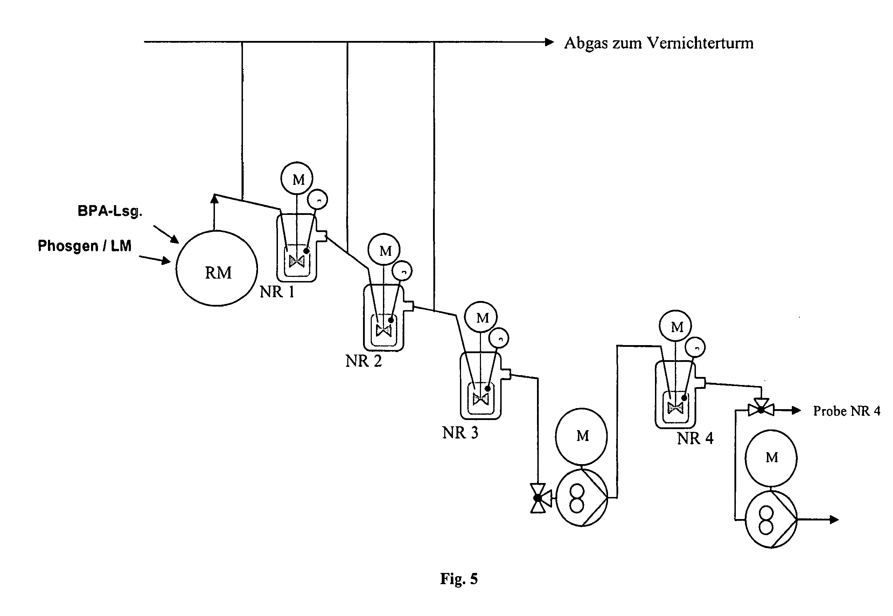

Aus der oder den Pumpen wird die oligocarbonathaltige Reaktionsmischung bzw. Chorameisensäurearylester- und/oder Diarylcarbonathaltige Reaktionsmischung in wenigstens einem Reaktor unter Zugabe von weiterer Alkalilauge, gegebenenfalls Kettenabbrecher(n) und gegebenenfalls wenigstens eines weiteren Katalysators zu Polycarbonaten aufkondensiert bzw. zu Diarylcarbonaten umgesetzt. In bevorzugten Ausführungsformen erfolgt diese Reaktion in einer Kaskade von mehreren hintereinander geschalteten Reaktoren. Als geeignete Reaktoren für die Reaktion kommen beliebige Reaktorausführungen, wie z.B. Rührkessel, Rohrreaktoren, Umpumpreaktoren, deren Kaskaden oder deren Kombinationen in Frage (vgl. z.B. Fig. 2 oder Fig. 5).

Zur Aufarbeitung des bei der Polycarbonatherstellung ausreagierten, höchstens noch Spuren, bevorzugt weniger als 2 ppm an Chlorkohlensäureestern enthaltende, mindestens zweiphasige Reaktionsgemisch lässt man zur Phasentrennung absitzen. Die wässrige alkalische Phase wird gegebenenfalls ganz oder teilweise zurück in die Polycarbonatsynthese als wässrige Phase geleitet oder aber der Abwasseraufarbeitung zugeführt, wo Lösungsmittel- und Katalysatoranteile abgetrennt und gegebenenfalls in die Polycarbonatsynthese rückgeführt werden. In einer anderen Variante der Aufarbeitung wird nach Abtrennung der organischen Verunreinigungen, insbesondere von Lösungsmitteln und Polymerresten, und gegebenenfalls nach der Einstellung eines bestimmten pH-Wertes, z.B. durch Natronlaugezugabe, das Salz abgetrennt, welches z. B. der Chloralkalielektrolyse zugeführt werden kann, während die wässrige Phase gegebenenfalls wieder der Polycarbonatsynthese zugeführt wird.

Zur Aufarbeitung des bei der Diarylcarbonatherstellung ausreagierten, höchstens noch Spuren, bevorzugt weniger als 2 ppm an Chlorameisensäurearylestern enthaltende, mindestens zweiphasige Reaktionsgemisch lässt man zur Phasentrennung absitzen. Die wässrige alkalische Phase wird gegebenenfalls ganz oder teilweise vereinigt mit Waschphasen der Abwasseraufarbeitung zugeführt, wo Lösungsmittel- und Katalysatoranteile durch Strippen abgetrennt und gegebenenfalls in die Diarylcarbonatsynthese rückgeführt werden. In einer anderen Variante der Aufarbeitung wird nach Abtrennung der organischen Verunreinigungen, insbesondere von Lösungsmitteln und Katalysatorresten, und gegebenenfalls nach der Einstellung eines bestimmten pH-Wertes, z.B. durch Salzsäurezugabe, die Salzlösung abgetrennt, welches z. B. der Chloralkali-Elektrolyse zugeführt werden kann, während die wässrige Phase gegebenenfalls wieder der Diarylcarbonatsynthese zugeführt wird.

Die organische, das Polycarbonat bzw. Diarylcarbonat enthaltende Phase kann anschließend zur Entfernung der Kontaminationen alkalischer, ionischer oder katalytischer Art auf verschiedene, dem Fachmann bekannte Weisen gereinigt werden.

Die organische Phase enthält in der Regel auch nach einem oder mehreren Absetzvorgängen, gegebenenfalls unterstützt durch Durchläufe durch Absetzkessel, Rührkessel, Coalescer oder Separatoren bzw. Kombinationen aus diesen Maßnahmen - wobei gegebenenfalls Wasser in jedem oder einigen Trennschritten unter Umständen unter Verwendung von aktiven oder passiven Mischorganen zudosiert werden kann - noch Anteile der wässrigen alkalischen Phase in feinen Tröpfchen sowie des oder der Katalysator(en). Nach dieser groben Abtrennung der alkalischen, wässrigen Phase kann die organische Phase ein oder mehrmals mit verdünnten Säuren, Mineral-, Carbon- Hydroxycarbon-und/oder Sulfonsäuren gewaschen werden. Bevorzugt sind wässrige Mineralsäuren insbesondere Salzsäure, phosphorige Säure, Phosphorsäure oder Mischungen dieser Säuren. Die Konzentration dieser Säuren sollte dabei vorzugsweise im Bereich 0,001 bis 50 Gew. %, bevorzugt 0,01 bis 5 Gew. % liegen. Weiterhin kann die organische Phase mit entsalztem oder destilliertem Wasser wiederholt gewaschen werden. Die Abtrennung der, gegebenenfalls mit Teilen der wässrigen Phase dispergierten, organischen Phase nach den einzelnen Waschschritten geschieht mittels Absetzkessel, Rührkessel, Coalescer oder Separatoren bzw. Kombinationen aus diesen Maßnahmen, wobei das Waschwasser zwischen den Waschschritten gegebenenfalls unter Verwendung von aktiven oder passiven Mischorganen zudosiert werden kann. Bei der Polycarbonatherstellung kann zwischen diesen Waschschritten oder auch nach der Wäsche können gegebenenfalls Säuren, vorzugsweise gelöst im Lösungsmittel welches der Polymerlösung zugrunde liegt, zugegeben werden. Bevorzugt werden hier Chlorwasserstoffgas, Phosphorsäure oder phosphorige Säure verwendet, die gegebenenfalls auch als Mischungen eingesetzt werden können. Die so erhaltene gereinigte Polycarbonatlösung sollte vorzugsweise nach dem letzten Trennvorgang nicht mehr als 5 Gew. %, bevorzugt weniger als 1 Gew. %, ganz besonders bevorzugt weniger als 0,5 Gew. % Wasser enthalten.

Die Isolierung des Diarylcarbonats aus der Lösung kann durch Verdampfen des Lösungsmittels mittels Temperatur, Vakuum oder eines erhitzten Schleppgases oder bevorzugt Destillation erfolgen.

Die nach dem erfindungsgemäßen Verfahren hergestellten Diarylcarbonate zeichnen sich durch besonders hohe Reinheit (GC >99,95%) und sehr gutes Umesterungsverhalten aus, so dass hieraus anschließend ein Polycarbonat in exzellenter Qualität hergestellt werden kann.

Die Verwendung der Diarylcarbonate zur Herstellung von aromatischen Oligo-/Polycarbonaten nach dem Schmelzumesterungsverfahren ist literaturbekannt und beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) oder der US-A- 5 340 905 beschrieben.

Die Isolierung des Polycarbonats aus der Lösung kann durch Verdampfen des Lösungsmittels mittels Temperatur, Vakuum oder eines erhitzten Schleppgases erfolgen. Andere Isolierungsmethoden sind beispielsweise Kristallisation und Fällung.

Erfolgt die Konzentrierung der Polycarbonatlösung und eventuell auch die Isolierung des Polycarbonats durch Abdestillieren des Lösungsmittels, gegebenenfalls durch Überhitzung und Entspannung, so spricht man von einem "Flash-Verfahren". Ein solches Verfahren ist dem Fachmann bekannt und beispielsweise in "Thermische Trennverfahren", VCH Verlagsanstalt 1988, S. 114 beschrieben. Wird statt dessen ein geheiztes Trägergas zusammen mit der einzudampfenden Lösung versprüht, so spricht man von einer "Sprühverdampfung/Sprühtrocknung", welche beispielhaft in Vauck, "Grundoperationen chemischer Verfahrenstechnik", Deutscher Verlag für Grundstoffindustrie 2000, 11.auflage, S. 690 beschrieben ist. Alle diese Verfahren sind in der Patentliteratur und in Lehrbüchern beschrieben und dem Fachmann geläufig.

Bei der Entfernung des Lösungsmittels durch Temperatur (Abdestillieren) bzw. dem technisch effektiveren Flash-Verfahren erhält man hochkonzentrierte Polycarbonatschmelzen. Beim Flashverfahren werden Polymerlösungen wiederholt unter leichtem Überdruck auf Temperaturen oberhalb des Siedepunktes unter Normaldruck erhitzt und diese, bezüglich des Normaldruckes, überhitzten Lösungen anschließend in ein Gefäß mit niedrigerem Druck, z.B. Normaldruck, entspannt. Es kann dabei von Vorteil sein, die Aufkonzentrationsstufen, oder anders ausgedrückt die Temperaturstufen der Überhitzung nicht zu groß werden zu lassen, sondern lieber ein zwei- bis vierstufiges Verfahren zu wählen.

Aus den so erhaltenen hochkonzentrierten Polycarbonatschmelzen können die Reste des Lösungsmittels entweder direkt aus der Schmelze mit Ausdampfextrudern (vgl. z.B. BE-A 866 991, EP-A 0 411 510, US-A 4 980 105, DE-A 33 32 065), Dünnschichtverdampfern (vgl. z.B. EP-A 0 267 025), Fallfilmverdampfern, Strangverdampfern oder durch Friktionskompaktierung (vgl. z.B. EP-A 0 460 450), gegebenenfalls auch unter Zusatz eines Schleppmittels, wie Stickstoff oder Kohlendioxid oder unter Verwendung von Vakuum (vgl. z.B. EP-A 0 039 96, EP-A 0 256 003, US-A 4 423 207), entfernt werden, alternativ auch durch anschließende Kristallisation (vgl. z.B. DE-A 34 29 960) und/oder Ausheizen der Reste des Lösungsmittels in der festen Phase (vgl. z.B. US-A 3 986 269, DE-A 20 53 876). Auch diese Verfahren und die hierzu erforderlichen Vorrichtungen sind in der Literatur beschrieben und dem Fachmann geläufig.

Polycarbonat-Granulate können - wenn möglich - durch direktes Abspinnen der Schmelze und anschließender Granulierung oder aber durch Verwendung von Austragsextrudern von denen in Luft oder unter Flüssigkeit, meist Wasser, abgesponnen wird, erhalten werden. Werden Extruder verwendet, so kann man der Polycarbonatschmelze vor dem Extruder, gegebenenfalls unter Einsatz von statischen Mischern oder durch Seitenextruder in diesem Extruder, Additive zusetzen.

Alternativ kann die Polycarbonatlösung einer Sprühverdampfung unterzogen werden. Bei einer Versprühung wird die Polycarbonatlösung gegebenenfalls nach Erhitzung entweder in ein Gefäß mit Unterdruck verdüst oder mittels einer Düse mit einem erhitzten Trägergas, z.B. Stickstoff, Argon oder Wasserdampf in ein Gefäß mit Normaldruck verdüst. In beiden Fällen erhält man in Abhängigkeit von der Konzentration der Polymerlösung Pulver (verdünnt) oder Flocken (konzentriert) des Polymers, aus dem gegebenenfalls auch die letzten Reste des Lösungsmittels wie oben entfernt werden müssen. Anschließend kann mittels eines Compoundierextruders und anschließender Abspinnung Granulat erhalten werden. Auch hier können Additive, wie oben beschrieben, in der Peripherie oder dem Extruder selbst, zugesetzt werden. Oftmals kann es erforderlich sein, vor der Extrusion aufgrund der geringen Schüttdichte der Pulver und Flocken einen Kompaktierungsschritt für das Polymerpulver zu durchlaufen.

Alternativ kann aus der gewaschenen und gegebenenfalls noch aufkonzentrierten Polycarbonatlösung durch Zugabe eines Nichtlösungsmittels für Polycarbonat das Polymer weitgehend ausgefällt werden. Die Nichtlösungsmittel wirken dabei als Fällungsmittel. Hierbei ist es vorteilhaft, erst eine geringe Menge des Nichtlösungsmittels zuzugeben und gegebenenfalls auch Wartezeiten zwischen den Zugaben der Chargen an Nichtlösungsmittel einzulegen. Es kann außerdem von Vorteil sein, verschiedene Nichtlösungsmittels einzusetzen. Verwendung als Fällungsmittel finden hier z.B. aliphatische oder cycloaliphatische Kohlenwasserstoffe, insbesondere Heptan, i-Octan oder Cyclohexan, Alkohole wie z.B. Methanol, Ethanol oder i-Propanol, Ketone, wie z.B. Aceton, oder Mischungen aus diesen. Bei der Fällung wird in der Regel die Polymerlösung langsam dem Fällungsmittel zugesetzt. Die so erhaltenen Polycarbonate werden wie bei der Sprühverdampfung beschrieben zu Granulat verarbeitet und gegebenenfalls additiviert.

Nach anderen Verfahren werden Fällungs- und Kristallisations-Produkte oder amorph erstarrte Produkte in feinkörniger Form durch Überleiten von Dämpfen eines oder mehrer Nichtlösungsmittel für Polycarbonat, unter gleichzeitiger Erhitzung unterhalb der Glastemperatur kristallisiert und weiter zu höheren Molekulargewichten aufkondensiert. Handelt es sich dabei um Oligomere gegebenenfalls mit unterschiedlichen Endgruppen (Phenolische und Kettenabbrecherenden) so spricht man von Festphasenaufkondensation.

Der Zusatz von Additiven dient der Verlängerung der Nutzungsdauer oder der verbesserung der Farbstabilität (Stabilisatoren), der Vereinfachung der Verarbeitung (z.B. Entformer, Fließhilfsmittel, Antistatika) oder der Anpassung der Polymereigenschaften an bestimmte Belastungen (Schlagzähmodifikatoren, wie Kautschuke; Flammschutzmittel, Farbmittel, Glasfasern).

Diese Additive können einzeln oder in beliebigen Mischungen, zusammen oder in mehreren verschiedenen Mischungen der Polymerschmelze zugesetzt werden. Dies kann direkt bei der Isolierung des Polymeren oder aber nach Aufschmelzen von Granulat in einem sogenannten Compoundierungsschritt erfolgen. Dabei können die Additive beziehungsweise deren Mischungen als Feststoff, vorzugsweise als Pulver, oder als Schmelze der Polymerschmelze zugesetzt werden. Eine andere Art der Dosierung ist die Verwendung von Masterbatches oder Mischungen von Masterbatches der Additive oder Additivmischungen.

Geeignete Additive sind beispielsweise beschrieben in "Additives for Plastics Handbook, John Murphy, Elsevier, Oxford 1999", im "Plastics Additives Handbook, Hans Zweifel, Hanser, München 2001".

Geeignete Antioxidantien bzw. Thermostabilisatoren sind beispielsweise:
Alkylierte Monophenole, Alkylthiomethylphenole, Hydrochinone und alkylierte Hydrochinone, Tocopherole, hydroxylierte Thiodiphenylether, Alkylidenbisphenole, O-, N- und S-Benzylverbindungen, hydroxybenzylierte Malonate, aromatische Hydroxybenzylverbindungen, Triazinverbindungen, Acylaminophenole, Ester von β-(3,5-Di-tert-butyl-4-hydroxyphenyl)propionsäure, Ester von β-(5-tert-Butyl-4-hydroxy-3-methylphenyl)propionsäure, Ester von β-(3,5-Dicyclohexyl-4-hydroxyphenyl)propionsäure, Ester von 3,5-Di-tert-butyl-4-hydroxyphenylessigsäure, Amide of β-(3,5-Di-tert-butyl-4-hydroxyphenyl)propionsäure, geeignete Thiosynergisten, sekundäre Antioxidantien, Phosphite, Phosphonite, Phosphonate und Phosphane, Benzofuranone oder Indolinone.

Bevorzugte Antioxidantien bzw. Thermostabilisatoren sind organische Phosphite, Phosphonate und Phosphane, meist solche bei denen die organischen Reste ganz oder teilweise aus gegebenenfalls substituierten aromatischen Resten bestehen.

Als Komplexierungsmittel für Schwermetalle und zur Neutralisation von Alkalispuren sind beispielsweise o- oder m-Phosphorsäuren, ganz oder teilweise veresterte Phosphate oder Phosphite geeignet.

Als Lichtschutzmittel (UV-Absorber) sind beispielsweise 2-(2'-Hydroxyphenyl)benzotriazole, 2-Hydroxybenzophenone, Ester von substituierten und unsubstituierten Benzoesäuren, Acrylate, sterisch gehinderte Amine, Oxamide, 2-(2-Hydroxyphenyl)-1,3,5-triazine oder substituierte Benztriazole geeignet, besonders bevorzugt sind substituierte Benztriazole.

Polypropylenglykole allein oder in Kombination mit z.B. Sulfonen oder Sulfonamiden als Stabilisatoren können gegen die Schädigung durch Gamma-Strahlen verwendet werden.

Diese und andere Stabilisatoren können einzeln oder in Kombinationen verwendet werden und in den genannten Formen dem Polymer zugesetzt werden.

Außerdem können Verarbeitungshilfsmittel wie Entformungsmittel, wie beispielsweise Derivate langkettiger Fettsäuren, zugesetzt werden. Bevorzugt sind z.B. Pentaerythrittetrastearat und Glycerinmonostearat. Sie werden allein oder im Gemisch vorzugsweise in einer Menge von 0,02 bis 1 Gew.-%, bezogen auf die Masse der Zusammensetzung eingesetzt. Geeignete flammhemmende Additive sind Phosphatester, d.h. Triphenylphosphat, Resorcindiphosphorsäureester, bromhaltige Verbindungen, wie bromierte Phosphorsäureester, bromierte Oligocarbonate und Polycarbonate, sowie bevorzugt Salze fluorierter organischer Sulfonsäuren. Geeignete Schlagzähmacher sind Butadienkautschuk mit aufgepfropftem Styrol-Acrylnitril oder Methylmethacrylat, Ethylen-Propylen-Kautschuke mit aufgepfropftem Maleinsäureanhydrid, Ethyl- und Butylacrylatkautschuke mit aufgepfropftem Methylmethacrylat oder Styrol-Acrylnitril, interpenetrierende Siloxan- und Acrylat-Netzwerke mit aufgepfropftem Methylmethacrylat oder Styrol-Acrylnitril.

Des Weiteren können Farbmittel, wie organische Farbstoffe oder Pigmente sowie anorganische Pigmente, IR-Absorber, einzeln, im Gemisch oder auch in Kombination mit Stabilisatoren, Glasfasern, Glas(hohl)kugeln oder anorganischen Füllstoffen zugesetzt werden.

Polycarbonatschmelzen, die durch Isolierung des Polymers oder durch Compoundierung erzeugt wurden, können durch einen Düsenkopf in Strangform abgesponnen, mit Gas, z.B. Luft oder Stickstoff, oder einer Kühlflüssigkeit, meist Wasser, abgekühlt und die erstarrten Stränge in handelsüblichen Granulatoren mit Schneiden die sich z.B. auf einer sich drehenden Walze befinden, in Luft, unter Schutzgas wie Stickstoff oder Argon oder unter Wasser granuliert werden. Je nach apparativer Ausführung entstehen dabei säulenförmige Granulate mit rundem oder elliptischem Querschnitt und rauher oder glatter Oberfläche. Die Schnittkanten können glatt sein oder glasartigen Bruch aufweisen mit ausgebrochenen Schnittkanten oder aber stehengebliebenen Resten an den Schnittkanten. Wünschenswert ist ein möglichst gleichmäßig geformtes Granulat mit möglichst wenig verbleibenden Überständen an den Schnittkanten. Weiterhin ist der Staubanteil im Granulat möglichst gering zu halten, bevorzugt unter 100 mg/kg Granulat. Der Durchmesser der Granulatkörner soll zwischen 0,5 mm und 10 mm, bevorzugt bei 1 bis 8 mm, besonders bevorzugt bei 3 bis 6 mm liegen. Während die Länge der Granulatkörner zwischen 1 bis 10 mm, bevorzugt zwischen 2 bis 8 mm und das Gewicht zwischen 10 und 50 mg bevorzugt zwischen 15 und 30 mg liegen sollte. Bevorzugt ist ein Granulat dessen Verhältnis von Durchmesser, bei elliptischem Querschnitt des mittleren Durchmessers, zur Länge 0,8 bis 1,2 beträgt, besonders bevorzugt eines mit dem Verhältnis von ca. 1. Diese Parameter unterliegen Größenverteilungen, bevorzugt sind möglichst enge Verteilungen, also möglichst gleichmäßig dimensionierte Granulate.

Abkühlung, Abspinnung, Granulierung und der anschließende Transport oder die Förderung des Granulates mit Gas oder Flüssigkeit, und die anschließende Lagerung, gegebenenfalls nach einem Durchmischungs- oder Homogenisierungsprozess, sind so zu gestalten, dass möglichst trotz der gegebenenfalls vorhandenen statischen Aufladung keine Verunreinigungen auf die Polymer-, Strang- oder Granulatoberfläche aufgebracht werden, wie beispielsweise Staub, Abrieb aus den Maschinen, aerosolartige Schmiermittel und andere Flüssigkeiten sowie Salze aus möglicherweise verwendeten Wasserbädern oder Kühlungen.

Die nach dem erfindungsgemäßen Verfahren hergestellten Polymere sind ebenfalls Gegenstand der vorliegenden Anmeldung.

Die nach dem erfindungsgemäßen Verfahren hergestellten Polymere weisen eine enge Molekulargewichtsverteilung auf, vorzugsweise sogar eine engere als die nach dem Stand der Technik mit dem Phasengrenzflächenverfahren hergestellten. Bevorzugt weisen die nach dem erfindungsgemäßen Verfahren hergestellten Polymere eine Uneinheitlichkeit U von 1,1 bis 1,6 (je nach Viskosität der hergestellten Polycarbonate) auf.

Die erfindungsgemäß hergestellten Polycarbonate eignen sich beispielsweise zur Herstellung von Extrudaten und Formkörpern, insbesondere solchen zur Verwendung im transparenten Bereich, ganz besonders im Bereich optischer Anwendungen wie z.B. Platten, Stegplatten, Verglasungen, Streuscheiben, Lampenabdeckungen oder optischer Datenspeicher, wie Audio-CD, CD-R(W), DVD, DVD-R(W), Minidiscs in ihren verschiedenen nur lesbaren oder aber einmalbeschreibbaren gegebenenfalls auch wiederholt beschreibbaren Ausführungsformen.

Weitere Anwendungen sind beispielsweise, ohne jedoch den Gegenstand der vorliegenden Erfindung einzuschränken:
- 1.: Sicherheitsscheiben, die bekanntlich in vielen Bereichen von Gebäuden, Fahrzeugen und Flugzeugen erforderlich sind, sowie als Schilde von Helmen.
- 2.: Folien
- 3.: Blaskörper (s.a. US-A 2 964 794), beispielsweise 1 bis 5 Gallon Wasserflaschen.
- 4.: Lichtdurchlässige Platten, wie Massivplatten oder insbesondere Hohlkammerplatten, beispielsweise zum Abdecken von Gebäuden wie Bahnhöfen, Gewächshäusern und Beleuchtungsanlagen.
- 5.: Optische Datenspeicher, wie Audio CD's, CD-R(W)'s, DCD's, DVD-R(W)'s, Minidiscs und den Folgeentwicklungen
- 6.: Ampelgehäuse oder Verkehrsschilder
- 7.: Schaumstoffe mit offener oder geschlossener gegebenenfalls bedruckbarer Oberfläche
- 8.: Fäden und Drähte (s.a. DE-A 11 37 167)
- 9.: Lichttechnische Anwendungen, gegebenenfalls unter Verwendung von Glasfasern für Anwendungen im transluzenten Bereich
- 10.: Transluzente Einstellungen mit einem Gehalt an Bariumsulfat und oder Titandioxid und oder Zirkoniumoxid bzw. organischen polymeren Acrylatkautschuken (EP-A 0 634 445, EP-A 0 269 324) zur Herstellung von lichtdurchlässigen und lichtstreuenden Formteilen.
- 11.: Präzisionsspritzgussteile, wie Halterungen, z.B. Linsenhalterungen; hier werden gegebenenfalls Polycarbonate mit Glasfasern und einem gegebenenfalls zusätzlichen Gehalt von 1-10 Gew. % Molybdändisulfid (bez. auf die gesamte Formmasse) verwendet.
- 12.: optische Geräteteile, insbesondere Linsen für Foto- und Filmkameras (DE-A 27 01 173).
- 13.: Lichtübertragungsträger, insbesondere Lichtleiterkabel (EP-A 0 089 801) und Beleuchtungsleisten
- 14.: Elektroisolierstoffe für elektrische Leiter und für Steckergehäuse und Steckverbinder sowie Kondensatoren.
- 15.: Mobiltelefongehäuse.
- 16.: Network interface devices
- 17.: Trägermaterialien für organische Fotoleiter
- 18.: Leuchten, Scheinwerferlampen, Streulichtscheiben oder innere Linsen.
- 19.: Medizinische Anwendungen wie Oxygenatoren, Dialysatoren.
- 20.: Lebensmittelanwendungen, wie Flaschen, Geschirr und Schokoladenformen.
- 21.: Anwendungen im Automobilbereich, wie Verglasungen oder in Form von Blends mit ABS als Stoßfänger.
- 22.: Sportartikel wie Slalomstangen, Skischuhschnallen.
- 23.: Haushaltsartikel, wie Küchenspülen, Waschbecken, Briefkästen
- 24.: Gehäuse, wie Elektroverteilerkästen
- 25.: Gehäuse für elektrische Geräte wie Zahnbürsten, Föne, Kaffeemaschinen, Werkzeugmaschinen, wie Bohr-, Fräs-, Hobelmaschinen und Sägen
- 26.: Waschmaschinen-Bullaugen
- 27.: Schutzbrillen, Sonnenbrillen, Korrekturbrillen bzw. deren Linsen.
- 28.: Lampenabdeckungen
- 29.: Verpackungsfolien
- 30.: Chip-Boxen, Chipträger, Boxen für Si-Wafer
- 31.: Sonstige Anwendungen wie Stallmasttüren oder Tierkäfige.

Überraschend konnte zudem mit dem erfindungsgemäßen Verfahren die Diphenol-Belastung bzw. Monophenol-Belastung des Abwassers reduziert werden.

Die Verwendung von Pumpen mit dreifacher Funktion als Mischaggregat, Reaktionaraum für den Oligomerisierungsschritt bzw. Chlorameisensäurearylester- und/oder Diarylcarbonatherstellungsschritt sowie Förderaggregat ist für das Phasengrenzflächenverfahren zur Herstellung von Polycarbonaten oder Diarylcarbonaten in der Literatur nicht beschrieben. Gegenstand der vorliegenden Erfindung ist daher weiterhin die Verwendung einer oder mehrerer Pumpe(n) zur kontinierlichen Herstellung von Polycarbonat bzw. Diarylcarbonat nach dem Phasengrenzflächenverfahren, wobei die Pumpe(n)
- nach dem Stator-Rotor-Prinzip arbeitet (arbeiten)
- thermostatisierbar ist (sind) und
- wenigstens jeweils einen Zugang für eine organische und eine wässrige Phase und gegebenenfalls weitere Zugänge für Kettenabbrecher, Verzweiger und/oder zusätzlich Alkalilauge, Katalysator sowie wenigstens einen Ausgang für die oligocarbonathaltige Mischung bzw. Chlorameisensäurearylester- und/oder Diarylcarbonathaltige Mischung aufweist (aufweisen),
dadurch gekennzeichnet, dass in dieser/diesen Pumpen sowohl die kontinuierliche Mischung der organischen und wässrigen Phase, wobei die organische Phase wenigstens ein für das Polycarbonat bzw. Diarylcarbonat geeignetes Lösungsmittel und ganz oder teilweise das Phosgen enthält und die wässrige Phase das oder die Diphenol(e) bzw. Monophenol(e), Wasser und Alkalilauge enthält, als auch die Umsetzung des oder der Diphenol(e) bzw. Monophenol(e) und Phosgen zu Oligocarbonaten bzw. Chlorameisensäurearylester- und/oder Diarylcarbonaten erfolgt.

Für diese Verwendung eignen sich die vorangehend für das erfindungsgemäße Verfahren bereits beschriebenen Pumpen. Insbesondere eignen sich hierfür Kreiselpumpen, besonders bevorzugt Peripheralradpumpen.

Die folgenden Beispiele dienen der beispielhaften Erläuterung der Erfindung und sind nicht als Beschränkung aufzufassen.

### Beispiele

In einem kontinuierlichen Phasengrenzflächenprozess wurden Polycarbonate mittels jeweils unterschiedlichen Reaktorkonzepten hergestellt. In den Versuchen wurde der Einfluss des erfindungsgemäßen Einsatzes der speziellen Pumpe gegenüber bekannten Reaktoren wie Umpumprekatoren oder Düsen bei ansonsten gleichen Reaktionseinstellungen untersucht.

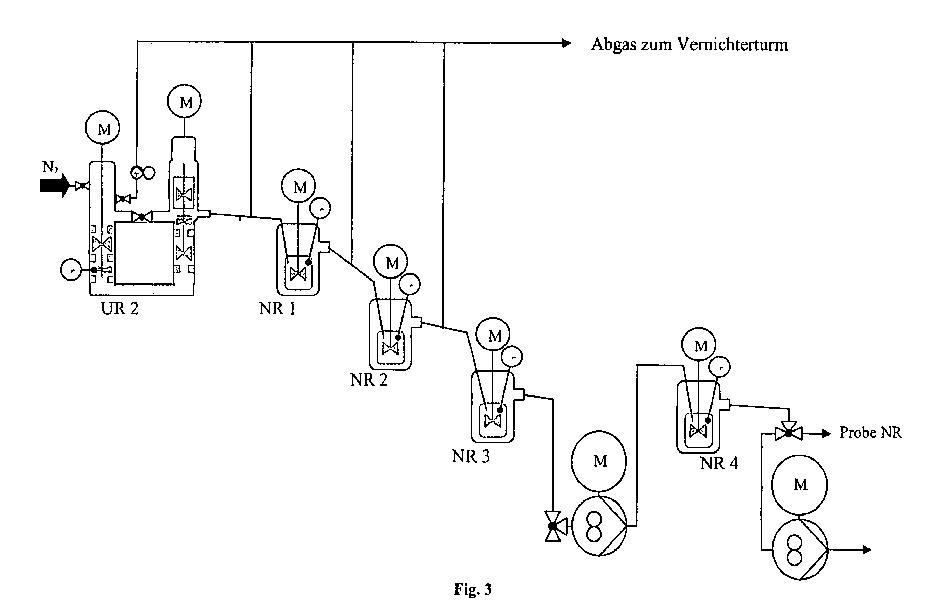

Die Reaktion wurde in allen Beispielen kontinuierlich in einer Emulsion aus Wasser und einem Lösungsmittelgemisch aus 50 Gew.-% Methylenchlorid und 50 Gew.-% Chlorbenzol durchgeführt. Als Kettenabbrecher wurde p-tert-Butylphenol (BUP) eingesetzt. Als Katalysator wurde n-Ethylpiperidin (EPP) eingesetzt. Die nach Abtrennen der wässrigen Phase nach Durchlaufen der Verweilkaskade aus den vier Nachreaktoren NR1 bis NR4 in Fig. 3 und 5 erhaltene Polycarbonatlösung wurde mit Salzsäure sauer gewaschen, und anschließend mittels Tellerseparatoren mit vollentsalztem Wasser neutral gewaschen. Die so gewaschenen Polycarbonatlösungen wurden nach Trocknung durch Abdampfen des Lösungsmittels aufkonzentriert. Eine vollständige Entfernung des restlichen Lösungsmittels im Vakuumtrockenschrank bei 100 °C führte zum Polycarbonat.

Die Durchführung erfolgte bei allen Beispielen mit folgenden Reaktionseinstellungen:
- Na-Bisphenolat-Lösung: Durchsatz 910,3 g/h (0,598 mol Bisphenol A/h), Na-Bisphenolat-Gehalt: 15,0 Gew.-% Na-Bisphenolat bezogen auf das Gesamtgewicht der Lösung
- Phosgen: 72,18 g/h (0,73 mol/h)
- Lösungsmittel: 737,7 g/h (Lösungsmittelgemisch aus 50 Gew.-% Methylenchlorid und 50 Gew.-% Chlorbenzol)
- Natronlauge: 113,9 g/h; NaOH-Gehalt: 44 Gew.-% NaOH, bezogen auf das Gesamtgewicht der Natronlauge, zum Lösen von 136,5 g Bisphenol A
- Natronlauge nach erstem Reaktor: 49,5 g/h; NaOH-Gehalt: 44 Gew.-% NaOH bezogen auf das Gesamtgewicht der Natronlauge
- Kettenabbrecher BUP: 3,594 g/h (in 140,2 g/h Lösungsmittelgemisch 50:50)
- Katalysator EPP: 0,677 g/h; (in 6,2 g/h Lösungsmittel 50:50)
- Phosgen / Bisphenol A: 122 mol-% Phosgen bezogen auf die Stoffmenge Bisphenol A
- EPP / Bisphenol A: 1,0 mol-% EPP bezogen auf die Stoffmenge Bisphenol A
- BUP / Bisphenol A: 4,0 mol-% BUP bezogen auf die Stoffmenge Bisphenol A
- Reaktionstemperatur: 34 °C
- Polycarbonatgehalt: 15,0 Gew.-% Polycarbonat, bezogen auf das Gesamtgewicht der Lösung, gelöst in vorangehend genanntem Lösungsmittel
- Zugabe des Kettenabbrechers nach dem ersten Reaktor

Die Zugabe des Kettenabbrechers erfolgte in allen Beispielen in der Weise, dass eine vergleichbare relative Viskosität (ηᵣₑₗ) für die Endprodukte erreicht wurde.

### Beispiel 1 (Vergleichsbeispiel):

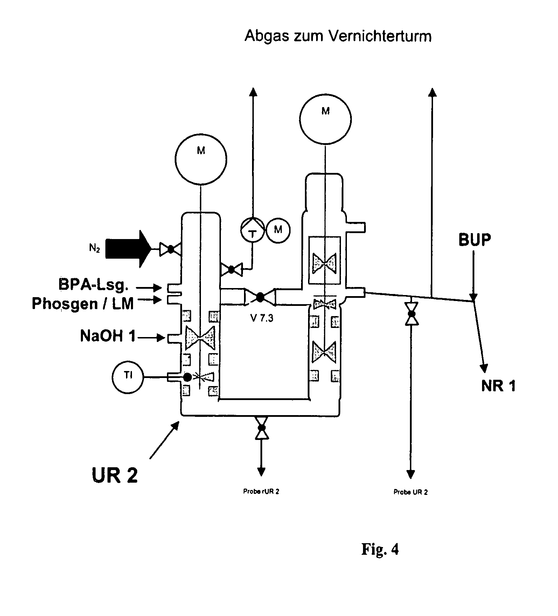

Gemäß Versuchaufbau in Fig. 3 wurde Polycarbonat hergestellt. Dabei wurde als erster Reaktor ein solcher Umpumpreaktor UR 2 gemäß Fig. 4 aus Glas verwendet, an den sich eine Verweilkaskade von vier Rührkesseln (Nachreaktoren NR1 bis NR4) zur Nachreaktion anschloss. In den einzelnen Reaktoren wurden die Rührelemente durch Motoren M angetrieben. Vor und nach dem letzten Nachreaktor NR4 befanden sich zudem zwei Zahnradpumpen mit Motor M. Na-Bisphenolat-Lösung, Phosgen in oben genannten Lösungsmittel und Natronlauge wurden in der oben genannten Menge kontinuierlich dem Umpumpreaktor zugeführt und durch Umpumpen bei geöffnetem Ventil V7.3 rückvermischt. Dabei wurde der verwendete Rührer im Umpumpreaktor mit 1000 Umdrehungen/min betrieben. Am Auslass des Umpumpreaktors wurde die Reaktionsmischung kontinuierlich aus dem Umpumpreaktor herausgeführt, mit Kettenabbrecher versetzt und in die in Fig. 3 gezeigte Verweilkaskade von vier Rührkesseln zur Nachreaktion geleitet. Der Katalysator wurde dem dritten Nachreaktor NR3 zugeführt.

### Beispiel 2 (Vergleichsbeispiel):

Es wurde der gleiche Versuchsaufbau verwendet wie in Beispiel 1, jedoch wurde im Umpumpreaktor UR 2 das Ventil V7.3 geschlossen, so dass eine Rückvermischung ausgeschlossen wurde. Dadurch wirkte der Umpumpreaktor UR 2 wie eine normale Düse.

### Beispiel 3 (erfindungsgemäß):

Anstelle des Umpumpreaktors UR 2 in Beispiel 1 wurde eine Peripheralradpumpe RM gemäß Fig. 1 verwendet. Ansonsten wurde der gleiche Versuchsaufbau verwendet wie in Beispiel 1 (vgl. Fig. 5). Na-Bisphenolat-Lösung, Phosgen in oben genannten Lösungsmittel und Natronlauge wurden in der oben genannten Menge kontinuierlich der Pumpe RM zugeführt und durch das mit Schaufeln versehende rotierende Peripheralrad vermischt. Am Auslass der Pumpe RM wurde die Reaktionsmischung kontinuierlich aus Innenraum der Pumpe herausgeführt, mit Kettenabbrecher versetzt und in die in Fig. 5 gezeigte Verweilkaskade von vier Rührkesseln zur Nachreaktion geleitet. Der Katalysator wurde dem dritten Nachreaktor NR3 zugeführt.

Tab. 1 zeigt die Ergebnisse hinsichtlich des erhaltenen Polycarbonats sowie die Belastung des resultierenden Abwassers mit Bisphenol A (BPA).

**Tab. 1:**

| **Beispiel** | **BPA-Gehalt im Abwasser [Gew.-ppm]** | **M_{w} [g/mol]** | **U** | **ηᵣₑₗ** |
|---|---|---|---|---|
| 1 | 238 | 19182 | 1,54 | 1,215 |
| 2 | 675 | 19111 | 1,69 | 1,214 |
| 3 | 48 | 19353 | 1,51 | 1,218 |

Die relative Lösungsviskosität ηᵣₑₗ wurde in Dichlormethan als Lösungsmittel bei einer Konzentration von 5 g/l und bei einer Temperatur von 25°C mit einem Ubbelohde-Viskosimeter bestimmt.

Der BPA-Gehalt im Abwasser wurde mittels HPLC und UV-Detektion (245 nm) nach Kalibrierung mit BPA ermittelt.

Die Ergebnisse zeigen eine deutliche Reduktion der Abwasserbelastung mit BPA bei erfindungsgemäßem Einsatz der speziellen Pumpe gegenüber der herkömmlichen Herstellung mittels Umpumpreaktor oder einfacher Düse. Im Falle der erfindungsgemäßen Durchführung des Phasengrenzflächenverfahrens ist daher eine Erhöhung des Phosgen- und Natronlaugezusatzes zur Vervollständigung der Umsetzung des Diphenols nicht erforderlich. Daher ist das erfindungsgemäß durchgeführte Verfahren zudem wirtschaftlicher als nach den bekannten Verfahren. Auch die Uneinheitlichkeit U, die ein Maß für die Breite der Molekulargewichtsverteilung des resultierenden Polycarbonats ist, konnte leicht gegenüber den Produkten erhältlich nach bekannten Verfahren verringert werden.

## Patentansprüche

1. Verfahren zur kontinuierlichen Herstellung von Polycarbonaten oder Copolycarbonaten bzw. Diarylcarbonaten nach dem Phasengrenzflächenverfahren aus wenigstens einem Diphenol bzw. Monophenol, Phosgen und wenigstens einem Katalysator gegebenenfalls in Gegenwart wenigstens eines Kettenabbrechers und/oder Verzweigers, **dadurch gekennzeichnet, dass**
(a) kontinuierlich eine Mischung der organischen und wässrigen Phase erzeugt wird, wobei die organische Phase wenigstens ein für das Polycarbonat bzw. Diarylcarbonat geeignetes Lösungsmittel und ganz oder teilweise das Phosgen enthält und die wässrige Phase das oder die Diphenol(e) bzw. Monophenol(e), Wasser und Alkalilauge enthält,
(b) eine Umsetzung des oder der Diphenol(e) bzw. Monophenol(e) und Phosgen zu Oligocarbonaten bzw. zu Chlorameisensäurearylestern oder Diarylcarbonaten oder einer Mischung aus Chlorameisensäurearylestern und Diarylcarbonaten erfolgt,
(c) die Oligocarbonate bzw. Chlorameisensäurearylester und/oder Diarylcarbonate anschließend in wenigstens einem Reaktor unter Zugabe von weiterer Alkalilauge, gegebenenfalls Kettenabbrecher(n) und gegebenenfalls wenigstens eines weiteren Katalysators umgesetzt werden,
**dadurch gekennzeichnet, dass** die kontinuierliche Mischung der organischen und wässrigen Phase unter (a) und die Umsetzung zu Oligocarbonaten bzw. Chlorameisensäurearylester und/oder Diarylcarbonate unter (b) in einer oder mehreren Pumpe(n) erfolgen, die
- nach dem Stator-Rotor-Prinzip arbeitet (arbeiten)
- thermostatisierbar ist (sind) und
- wenigstens jeweils einen Zugang für die organische und die wässrige Phase und gegebenenfalls weitere Zugänge für Katalysator, Kettenabbrecher, Verzweiger und/oder zusätzlich Alkalilauge sowie wenigstens einen Ausgang für die oligocarbonathaltige bzw. Chlorameisensäurearylester-Diarylcarbonat Mischung aufweist (aufweisen).

2. Verfahren gemäß Anspruch 1 **dadurch gekennzeichnet, dass** im Schritt (c) wenigstens ein weiterer Katalysator eingesetzt wird.

3. Verfahren gemäß Anspruch 1 oder 2, **dadurch gekennzeichnet, dass** die Pumpe(n) weitere Zugänge für zusätzlich Alkalilauge sowie wenigstens einen Ausgang für die Chlorameisensäurearylester und/oder Diarylcarbonat-haltige Mischung aufweist (aufweisen)

4. Verfahren gemäß einem der Ansprüche 1 bis 3, **dadurch gekennzeichnet, dass** die Pumpe(n) einen oder mehrere Rotoren aufweist (aufweisen).

5. Verfahren gemäß einem der Ansprüche 1 bis 4, **dadurch gekennzeichnet, dass** die Pumpe(n) auf eine Temperatur von -5°C bis 100°C, bevorzugt von 15°C bis 80°C, besonders bevorzugt von 25°C bis 65°C thermostatisierbar ist.

6. Verfahren gemäß einem der Ansprüche 1 bis 5, **dadurch gekennzeichnet, dass** als Diphenole solche der allgemeinen Formel
HO-Z-OH
eingesetzt werden, worin Z ein divalenter organischer Rest mit 6 bis 30 Kohlenstoffatomen ist, der eine oder mehrere aromatische Gruppen enthält.

7. Verfahren gemäß Anspruch 6, **dadurch gekennzeichnet, dass** als Diphenol(e) Hydrochinon, Resorcin, Dihydroxydiphenyle, Bis-(hydroxyphenyl)-alkane, Bis(hydroxy-phenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α,α'-Bis-(hydroxyphenyl)-diisopropylbenzole, Bis-(hydroxyphenyl)phtalimidine oder deren alkylierte, kernalkylierte und kernhalogenierte Verbindungen eingesetzt werden.

8. Verfahren gemäß Anspruch 6, **dadurch gekennzeichnet, dass** als Diphenol(e) 4,4'-Dihydroxydiphenyl, 2,2-Bis-(4-hydroxyphenyl)-1-phenyl-propan, 1,1-Bis-(4-hydroxyphenyl)-phenyl-ethan, 2,2-Bis-(4-hydroxyphenyl)propan (Bisphenol A, BPA), 2,4-Bis-(4-hydroxyphenyl)-2-methylbutan, 1,3-Bis-[2-(4-hydroxyphenyl)-2-propyl]benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol, 2-Hydrocarbyl-3,3-bis(4-hydroxyphenyl)phthalimidin, 3,3-Bis(4-hydroxyphenyl)-1-phenyl-1H-indol-2-on, 2,2-Bis(4-hydroxyphenyl)-1-phenyl-1H-indol-2-on, 3,3-Bis(4-hydroxyphenyl)-1-methyl-1H-indol-2-on, 2,2-Bis(4-hydroxyphenyl)-1-methyl-1H-indol-2-on, 3,3-Bis(4-hydroxyphenyl)-N-methyl-phthalimidin, 3,3-Bis(4-hydroxyphenyl)-N-phenyl-phthalimidin und 1,1-Bis-(4-hydroxyphenyl)-3,3,5-trimethylcyclohexan (Bisphenol TMC) eingesetzt werden.

9. Verfahren gemäß einem der Ansprüche 1 bis 5, **dadurch gekennzeichnet, dass** als Monophenol(e) Verbindungen der Formel (I) eingesetzt werden: 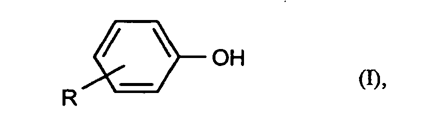 in welcher R = Wasserstoff, Halogen oder ein verzweigter oder unverzweigter C₁- bis C₉-Alkylrest oder Alkoxycarbonylrest ist.

10. Verfahren gemäß Anspruch 9, **dadurch gekennzeichnet, dass** als Monophenol(e) Phenol, Kresole, p-tert.-Butylphenol, p-Cumylphenol, p-n-Octylphenol, p-iso-Octylphenol, p-n-Nonylphenol und p-iso-Nonylphenol, p-Chlorphenol, 2,4-Dichlorphenol, p-Bromphenol und 2,4,6-Tribromphenol oder Salicylsäuremethylester oder eine Mischung dieser Verbindungen eingesetzt werden.

11. Verfahren gemäß einem der Ansprüche 1 bis 10, **dadurch gekennzeichnet, dass** es sich bei der Pumpe bzw. den Pumpen, die in (a) und/oder (b) eingesetzt werden, um eine Kreiselpumpe handelt.

12. Verfahren gemäß Anspruch 11 **dadurch gekennzeichnet, dass** es sich bei der Kreiselpumpe um eine Peripheralradpumpe handelt.

13. Verfahren gemäß wenigstens einem der Ansprüche 1 bis 12, **dadurch gekennzeichnet, dass** der die Pumpe(n) nach dem Ein- oder Mehrkammerprinzip aufgebaut ist (sind).

14. Verwendung einer oder mehrerer Pumpe(n) zur kontinierlichen Herstellung von Polycarbonat bzw. Diarylcarbonat nach dem Phasengrenzflächenverfahren, wobei die Pumpe(n)
- nach dem Stator-Rotor-Prinzip arbeitet (arbeiten)
- thermostatisierbar ist (sind) und
- wenigstens jeweils einen Zugang für eine organische und eine wässrige Phase aufweist.

15. Verwendung einer oder mehrerer Pumpe(n) gemäß Anspruch 14 **dadurch gekennzeichnet, dass** weitere Zugänge für Kettenabbrecher, Verzweiger und/oder zusätzlich Alkalilauge, Katalysator sowie wenigstens einen Ausgang für die oligocarbonathaltige Mischung bzw. Chlorameisensäurearylester- und/oder Diarylcarbonathaltige Mischung vorhanden sind.

16. Verwendung einer oder mehrerer Pumpe(n) gemäß Anspruch 15 **dadurch gekennzeichnet, dass** in dieser/diesen Pumpen sowohl die kontinuierliche Mischung der organischen und wässrigen Phase, wobei die organische Phase wenigstens ein für das Polycarbonat bzw. Diarylcarbonat geeignetes Lösungsmittel und ganz oder teilweise das Phosgen enthält und die wässrige Phase das oder die Diphenol(e) bzw. Monophenol(e), Wasser und Alkalilauge enthält, als auch die Umsetzung des oder der Diphenol(e) bzw. Monophenol(e) und Phosgen zu Oligocarbonaten bzw. Chlorameisensäurearylester- und/oder Diarylcarbonaten erfolgt.

17. Verwendung einer oder mehrerer Pumpe(n) gemäß einem der Ansprüche 13 bis 16, **dadurch gekennzeichnet, dass** es sich bei der oder den Pumpe(n) um Kreiselpumpen handelt.

18. Verwendung einer oder mehrerer Pumpe(n) gemäß Anspruch 17, **dadurch gekennzeichnet, dass** es sich bei der oder den Kreiselpumpe(n) um Peripheralradpumpen handelt.

## Claims

1. Process for the continuous preparation of polycarbonates or copolycarbonates or diaryl carbonates by the phase boundary process from at least one diphenol or monophenol, phosgene and at least one catalyst, optionally in the presence of at least one chain terminator and/or branching agent, **characterized in that**
(a) a mixture of the organic and aqueous phase is produced continuously, the organic phase containing at least one solvent suitable for the polycarbonate or diaryl carbonate and all or some of the phosgene and the aqueous phase containing the diphenol(s) or monophenol(s), water and alkali solution,
(b) a reaction of the diphenol(s) or monophenol(s) and phosgene takes place to give oligocarbonates or to give aryl chloroformates or diaryl carbonates or a mixture of aryl chloroformates and diaryl carbonates,
(c) the oligocarbonates or aryl chloroformates and/or diaryl carbonates are then reacted in at least one reactor with addition of further alkali solution, optionally chain terminator(s) and optionally at least one further catalyst,
**characterized in that** the continuous mixing of the organic and aqueous phase under (a) and the reaction to give oligocarbonates or aryl chloroformates and/or diaryl carbonates under (b) are effected in one or more pump(s) which
- operates or operate according to the stator-rotor principle
- is or are thermostatable and
- has or have in each case at least one entrance for the organic and the aqueous phase and optionally further entrances for a catalyst, chain terminator, branching agent and/or additionally alkali solution and at least one exit for the oligocarbonate-containing or aryl chloroformate/diaryl carbonate mixture.

2. Process according to Claim 1, **characterized in that** at least one further catalyst is used in step (c).

3. Process according to Claim 1 or 2, **characterized in that** the pump(s) has (have) further entrances for additionally alkali solution and at least one exit for the aryl chloroformate and/or diaryl carbonate-containing mixture.

4. Process according to one of Claims 1 to 3, **characterized in that** the pump(s) has (have) one or more rotors.

5. Process according to one of Claims 1 to 4, **characterized in that** the pump(s) is thermostatable to a temperature of -5°C to 100°C, preferably of 15°C to 80°C, particularly preferably of 25°C to 65°C.

6. Process according to one of Claims I to 5, **characterized in that** the diphenols used are those of the general formula
HO-Z-OH,
in which Z is a divalent organic radical having 6 to 30 carbon atoms, which contains one or more aromatic groups.

7. Process according to Claim 6, **characterized in that** hydroquinone, resorcinol, dihydroxybiphenyls, bis(hydroxyphenyl)alkanes, bis(hydroxyphenyl)cycloalkanes, bis(hydroxyphenyl)sulphides, bis(hydroxyphenyl)ethers, bis(hydroxyphenyl)ketones, bis(hydroxyphenyl)sulphones, bis(hydroxyphenyl)sulphoxides, α,α'-bis(hydroxyphenyl)diisopropylbenzenes, bis(hydroxyphenyl)phthalimidines or the compounds thereof which are alkylated, alkylated on the nucleus and halogenated on the nucleus are used as diphenol(s).

8. Process according to Claim 6, **characterized in that** 4,4'-dihydroxybiphenyl, 2,2-bis(4-hydroxyphenyl)-1-phenylpropane, 1,1-bis(4-hydroxyphenyl)phenylethane, 2,2-bis(4-hydroxyphenyl)propane (bisphenol A (BPA)), 2,4-bis(4-hydroxyphenyl)-2-methylbutane, 1,3-bis[2-(4-hydroxyphenyl)-2-propyl]benzene (bisphenol M), 2,2-bis(3-methyl-4-hydroxyphenyl)propane, bis(3,5-dimethyl-4-hydroxyphenyl)methane, 2,2-bis-(3,5-dimethyl-4-hydroxyphenyl)propane, bis(3,5-dimethyl-4-hydroxyphenyl) sulphone, 2,4-bis(3,5-dimethyl-4-hydroxyphenyl)-2-methylbutane, 1,3-bis[2-(3,5-dimethyl-4-hydroxyphenyl)-2-propyl]benzene, 2-hydroxycarbyl-3,3-bis(4-hydroxyphenyl)-phthalimidine, 3,3-bis(4-hydroxyphenyl)-1-phenyl-1H-indol-2-one, 2,2-bis(4-hydroxyphenyl)-1-phenyl-1H-indol-2-one, 3,3-bis(4-hydroxyphenyl)-1-methyl-1H-indol-2-one, 2,2-bis(4-hydroxyphenyl)-1-methyl-1H-indol-2-one, 3,3-bis(4-hydroxyphenyl)-N-methylphthalimidine, 3,3-bis(4-hydroxyphenyl)-N-phenylphthalimidine and 1,1-bis-(4-hydroxyphenyl)-3,3,5-trimethylcyclohexane (bisphenol TMC) are used as diphenol(s).

9. Process according to one of Claims 1 to 5, **characterized in that** the monophenol(s) used are compounds of the formula (I): 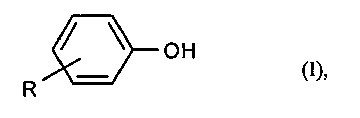 in which R is hydrogen, halogen or a branched or straight-chain C₁- to C₉-alkyl radical or alkoxycarbonyl radical.

10. Process according to Claim 9, **characterized in that** the monophenol(s) used are phenol, cresols, p-tert-butylphenol, p-cumylphenol, p-n-octylphenol, p-isooctylphenol, p-n-nonylphenol and p-isononylphenol, p-chlorophenol, 2,4-dichlorophenol, p-bromophenol and 2,4,6-tribromophenol or methyl salicylate or a mixture of these compounds.

11. Process according to one of Claims 1 to 10, **characterized in that** the pump or pumps which are used in (a) and/or (b) is or are a centrifugal pump.

12. Process according to Claim 11, **characterized in that** the centrifugal pump is a peripheral wheel pump.

13. Process according to at least one of Claims 1 to 12, **characterized in that** the pump(s) is (are) designed according to the one-chamber or multichamber principle.

14. Use of one or more pump(s) for the continuous preparation of polycarbonate or diaryl carbonate by the phase boundary process, the pump(s)
- operating according to the stator-rotor principle
- being thermostatable and
- having in each case at least one entrance for an organic and an aqueous phase.

15. Use of one or more pump(s) according to Claim 14, **characterized in that** further entrances for chain terminators, branching agents and/or additionally alkali solution, catalyst and at least one exit for the oligocarbonate-containing mixture or aryl chloroformate- and/or diaryl carbonate-containing mixture are present.

16. Use of one or more pump(s) according to Claim 15, **characterized in that** both the continuous mixing of the organic and aqueous phase, the organic phase containing at least one solvent suitable for the polycarbonate or diaryl carbonate and some or all of the phosgene and the aqueous phase containing the diphenol(s) or monophenol(s), water and alkali solution, and the reaction of the diphenol(s) or monophenol(s) and phosgene to give oligocarbonates or aryl chloroformates and/or diaryl carbonates are effected in this pump/these pumps.

17. Use of one or more pump(s) according to one of Claims 13 to 16, **characterized in that** the pump(s) are centrifugal pumps.

18. Use of one or more pump(s) according to Claim 17, **characterized in that** the centrifugal pump(s) are peripheral wheel pumps.

## Revendications

1. Procédé pour la production continue de polycarbonates ou de copolycarbonates ou, selon le cas, de diarylcarbonates selon le procédé interfacial à partir d'au moins un diphénol ou, selon le cas, un monophénol, de phosgène et d'au moins un catalyseur le cas échéant en présence d'au moins un agent d'interruption de chaîne et/ou agent de ramification, **caractérisé en ce que**
(a) on produit en continu un mélange de la phase organique et de la phase aqueuse, la phase organique contenant au moins un solvant approprié pour le polycarbonate ou, selon le cas, le diarylcarbonate et tout le phosgène ou une partie de celui-ci et la phase aqueuse contenant le ou les diphénol(s) ou, selon le cas, monophénol(s), de l'eau et de la lessive alcaline,
(b) on réalise une transformation du ou des diphénol(s) ou, selon le cas, monophénol(s) et du phosgène en oligocarbonates ou, selon le cas, en esters aryliques de l'acide chloroformique ou en diarylcarbonates ou en un mélange d'esters aryliques de l'acide chloroformique et de diarylcarbonates,
(c) on transforme ensuite les oligocarbonates ou, selon le cas, les esters aryliques de l'acide chloroformique et/ou les diarylcarbonates dans au moins un réacteur avec addition de lessive alcaline supplémentaire, le cas échéant un ou des agent(s) d'interruption de chaîne et le cas échéant au moins un autre catalyseur,
**caractérisé en ce que** le mélange continu de la phase organique et de la phase aqueuse sous (a) et la transformation en oligocarbonates ou, selon le cas, en esters aryliques de l'acide chloroformique et/ou en diarylcarbonates sous (b) sont réalisés dans une ou plusieurs pompe(s), qui
- fonctionne(fonctionnent) selon le principe de stator-rotor
- est(sont) thermostatisable(s) et
- présente(présentent) au moins une entrée pour la phase organique et la phase aqueuse et le cas échéant d'autres entrées pour le catalyseur, l'agent d'interruption de chaîne, l'agent de ramification et/ou de la lessive alcaline supplémentaire ainsi qu'au moins une sortie pour le mélange contenant les oligocarbonates ou, selon le cas, les esters aryliques de l'acide chloroformique-le diarylcarbonate.

2. Procédé selon la revendication 1, **caractérisé en ce qu'**au moins un autre catalyseur est utilisé dans l'étape (c).

3. Procédé selon la revendication 1 ou 2, **caractérisé en ce que** la(les) pompe(s) présente(présentent) d'autres entrées pour de la lessive alcaline supplémentaire ainsi qu'au moins une sortie pour le mélange contenant les esters aryliques de l'acide chloroformique et/ou les diarylcarbonates.

4. Procédé selon l'une quelconque des revendications 1 à 3, **caractérisé en ce que** la(les) pompe(s) présente(nt) un ou plusieurs rotors.

5. Procédé selon l'une quelconque des revendications 1 à 4, **caractérisé en ce que** la(les) pompe(s) est(sont) thermostatisable(s) à une température de -5 à 100°C, de préférence de 15 à 80°C et de manière particulièrement préférée de 25 à 65°C.

6. Procédé selon l'une quelconque des revendications 1 à 5, **caractérisé en ce qu'**on utilise comme diphénols ceux de formule générale
HO-Z-OH
où Z représente un radical organique divalent comprenant 6 à 30 atomes de carbone, qui contient un ou plusieurs groupes aromatiques.

7. Procédé selon la revendication 6, **caractérisé en ce qu'**on utilise comme diphénol(s) l'hydroquinone, le résorcinol, les dihydroxydiphényles, les bis-(hydroxyphényl)-alcanes, les bis(hydroxyphényl)-cycloalcanes, les bis-(hydroxyphényl)-sulfures, les bis-(hydroxyphényl)-éthers, les bis-(hydroxyphényl)-cétones, les bis-(hydroxyphényl)-sulfones, les bis-(hydroxyphényl)-sulfoxydes, les α,α'-bis-(hydroxyphényl)-diisopropylbenzènes, les bis-(hydroxyphényl)phtalimidines ou leurs composés alkylés, à noyau alkylé et à noyau halogéné.

8. Procédé selon la revendication 6, **caractérisé en ce qu'**on utilise comme diphénol(s) le 4,4'-dihydroxydiphényle, le 2,2-bis-(4-hydroxyphényl)-1-phénylpropane, le 1,1-bis-(4-hydroxyphényl)-phényléthane, le 2,2-bis-(4-hydroxyphényl)propane (Bisphénol A, BPA), le 2,4-bis-(4-hydroxyphényl)-2-méthylbutane, le 1,3-bis-[2-(4-hydroxyphényl)-2-propyl]benzène (Bisphénol M), le 2,2-bis-(3-méthyl-4-hydroxyphényl)-propane, le bis-(3,5-diméthyl-4-hydroxyphényl)-méthane, le 2,2-bis-(3,5-diméthyl-4-hydroxyphényl)-propane, la bis-(3,5-diméthyl-4-hydroxyphényl)-sulfone, le 2,4-bis-(3,5-diméthyl-4-hydroxyphényl)-2-méthylbutane, le 1,3-bis-[2-(3,5-diméthyl-4-hydroxyphényl)-2-propyl]-benzêne, la 2-hydrocarbyl-3,3-bis(4-hydroxyphényl)phtalimidine, la 3,3-bis(4-hydroxyphényl)-1-phényl-1H-indol-2-one, la 2,2-bis(4-hydroxyphényl)-1-phényl-1H-indol-2-one, la 3,3-bis(4-hydroxyphényl)-1-méthyl-1H-indol-2-one, le 2,2-bis(4-hydroxyphényl)-1-méthyl-1H-indol-2-one, la 3,3-bis(4-hydroxyphényl)-N-méthylphtalimidine, la 3,3-bis(4-hydroxyphényl)-N-phénylphtalimidine et le 1,1-bis-(4-hydroxyphényl)-3,3,5-triméthylcyclohexane (Bisphénol TMC).

9. Procédé selon l'une quelconque des revendications 1 à 5, **caractérisé en ce qu'**on utilise comme monophénol(s) des composés de formule (I), 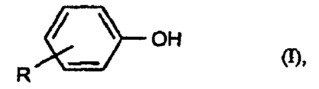 dans laquelle R = hydrogène, halogène ou un radical C₁-C₉-alkyle ramifié ou non ramifié ou un radical alcoxycarbonyle.

10. Procédé selon la revendication 9, **caractérisé en ce qu'**on utilise comme monophénol(s) le phénol, les crésols, le p-tert-butylphénol, le p-cumylphénol, le p-n-octylphénol, le p-iso-octylphénol, le p-n-nonylphénol et le p-iso-nonylphénol, le p-chlorophénol, le 2,4-dichlorophénol, le p-bromophénol et le 2,4,6-tribromophénol ou l'ester méthylique de l'acide salicylique ou un mélange de ces composés.

11. Procédé selon l'une quelconque des revendications 1 à 10, **caractérisé en ce qu'**il s'agit pour la ou, selon le cas, les pompe(s) qui est(sont) utilisée(s) dans (a) et/ou (b), d'une pompe centrifuge.

12. Procédé selon la revendication 11, **caractérisé en ce qu'**il s'agit, pour la pompe centrifuge, d'une pompe à roue périphérique.

13. Procédé selon au moins l'une quelconque des revendications 1 à 12, **caractérisé en ce que** la(les) pompe (s) est(sont) construite (s) selon le principe à une ou plusieurs chambres.

14. Utilisation d'une ou de plusieurs pompe(s) pour la production continue de polycarbonate ou, selon le cas, de diarylcarbonate selon le procédé interfacial, où la(les) pompe(s)
- fonctionne(fonctionnent) selon le principe de stator-rotor
- est(sont) thermostatisable(s) et
- présente(nt) au moins une entrée pour une phase organique et une phase aqueuse.

15. Utilisation d'une ou de plusieurs pompe(s) selon la revendication 14, **caractérisée en ce que** d'autres entrées pour l'agent d'interruption de chaîne, l'agent de ramification et/ou de la lessive alcaline supplémentaire, un catalyseur ainsi qu'au moins une sortie pour le mélange contenant des oligocarbonates ou, selon le cas, des esters de l'acide chloroformique et/ou des diarylcarbonates sont présentes.

16. Utilisation d'une ou de plusieurs pompe(s) selon la revendication 15, **caractérisée en ce qu'**on réalise dans cette(ces) pompe(s) le mélange continu de la phase organique et de la phase aqueuse, la phase organique contenant au moins un solvant approprié pour le polycarbonate ou, selon le cas, le diarylcarbonate et tout le phosgène ou une partie de celui-ci et la phase aqueuse contenant le ou les diphénol(s) ou, selon le cas, monophénol(s), de l'eau et de la lessive alcaline, ainsi que la transformation du ou des diphénol(s) ou, selon le cas, monophénol(s) et du phosgène en oligocarbonates ou, selon le cas, esters aryliques de l'acide chloroformique et/ou diarylcarbonates.

17. Utilisation d'une ou de plusieurs pompe(s) selon l'une quelconque des revendications 13 à 16, **caractérisée en ce qu'**il s'agit, pour la ou les pompe(s), de pompe(s) centrifuge(s).

18. Utilisation d'une ou de plusieurs pompe(s) selon la revendication 17, **caractérisée en ce qu'**il s'agit, pour la ou les pompe(s) centrifuge(s), de pompes à roue périphérique.
